(19) Europäisches Patentamt
European Patent Office
Office européen des brevets

(11) Veröffentlichungsnummer: **0 407 344 A2**

(12) **EUROPÄISCHE PATENTANMELDUNG**

(21) Anmeldenummer: **90810486.2**

(22) Anmeldetag: **28.06.90**

(51) Int. Cl.5: **A61K 31/66**, A61L 15/44

(30) Priorität: **07.07.89 CH 2532/89**

(43) Veröffentlichungstag der Anmeldung:
**09.01.91 Patentblatt 91/02**

(84) Benannte Vertragsstaaten:
**AT BE CH DE DK ES FR GB GR IT LI LU NL SE**

(71) Anmelder: **CIBA-GEIGY AG**
**Klybeckstrasse 141**
**CH-4002 Basel(CH)**

(72) Erfinder: **Ferrini, Pier Giorgio, Dr.**
**Im Rehwechsel 22**
**CH-4102 Binningen(CH)**
Erfinder: **Voellmy, Carlo, Dr.**
**Waldhofstrasse 44**
**CH-4310 Rheinfelden(CH)**
Erfinder: **Stahl, Peter Heinrich, Dr.**
**Lerchenstrasse 28**
**D-7800 Freiburg(DE)**
Erfinder: **Green, Jonathan, Dr.**
**Waldstrasse 12**
**CH-4144 Arlesheim(CH)**

(54) **Topisch applizierbare pharmazeutische Zubereitungen.**

(57) Die Erfindung betrifft topisch applizierbare pharmazeutische Präparate enthantend pharmazeutisch verwendbare Methandiphosphonsäure-Derivate der Formel

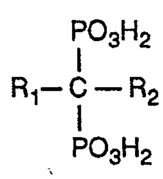

(I)

und ihre Salze in Betracht worin $R_1$ und $R_2$ jeweils Halogen bedeuten, oder worin $R_1$ Wasserstoff ist und $R_2$ Ar-S- oder $Het_1$-NH- bedeutet und Ar gegebenenfalls substituiertes Phenyl bedeutet und $Het_1$ gegebenenfalls substituiertes, monocyclisches, 5-oder 6-gliedriges Monoaza-, Diaza-oder Thiazaaryl bedeutet, welches über ein Ringkohlenstoffatom gebunden ist, oder worin $R_1$ Wasserstoff oder Hydroxy bedeutet und $R_2$ für -A-$R_3$ steht, worin A Alkylen bedeutet, und einerseits $R_3$ für $Het_2$ steht, das über ein Ringkohlenstoff- oder Ringstickstoffatom gebundenes $Het_1$ bedeutet, oder andererseits $R_3$ Wasserstoff oder gegebenenfalls durch Alkyl, Cycloalkyl, Ar-Alkyl, Ar-O-Alkyl, Ar-S-Alkyl oder $Het_1$-Alkyl unabhängig voneinander mono-oder disubstituiertes oder durch gegebenenfalls Ar enthaltendes Alkylen disubstituiertes Amino bedeutet, wobei zwei Alkylenkohlenstoffatome zusätzlich über Alkylen miteinander verknüpft sein können und Ar bzw. $Het_1$ jeweils die vorstehend angegebenen Bedeutungen hat, mit der Massgabe, dass, wenn $R_2$ für -A-$R_3$ steht, A Ethylen bedeutet und $R_3$ gegebenenfalls durch $C_1$-$C_3$-Alkyl substituiertes Amino bedeutet, $R_1$ von Hydroxy verschieden ist.

EP 0 407 344 A2

## TOPISCH APPLIZIERBARE PHARMAZEUTISCHE ZUBEREITUNGEN

Vertreter einer Klasse von synthetischen Methandiphosphonsäure-Derivaten werden bekanntermassen beispielsweise zur Behandlung von osteolytischen Knochenmetastasen und Hypercalcämie eingesetzt, da sie in der Lage sind, das Wachstum und die Zersetzung von Hydroxyapatit zu hemmen. Somit stoppen diese Verbindungen die Knochenresorption, indem sie sich spontan an das Hydroxyapatit des Knochens binden, so dass z.B. Osteoklasten keine Hydroxyapatitkristalle mehr abspalten können. Verbindungen dieser Substanzklasse werden beispielsweise in der DE-OS 2,405,254 beschrieben.

Es ist bekannt, dass entsprechende Methandiphosphonsäure-Derivate nach oraler Verabreichung nur in geringem Masse resorbiert werden, vor allem bei gleichzeitiger Nahrungszufuhr. Um den gewünschten therapeutischen Effekt zu erreichen, müssen dementsprechend höhere Dosierungen verabreicht werden.

Wegen der Bedeutung dieser Substanzklasse für die Behandlung beispielsweise von Osteoporose, Morbus Paget, Morbus Bechterew sowie Knochenmetastasenbildungen liegt das Ziel vielfacher Bemühungen darin, eine pharmazeutische Applikationsform bereitzustellen, aus der unabhängig von einer Nahrüngsmittelzufuhr die Wirkstoffe leicht resorbiert werden können.

Die erfindungsgemäss eingesetzten Methandiphosphonsäure-Derivate, die zwei dissoziierbare Säuregruppen sowie ein basisches Zentrum aufweisen, liegen bekanntermassen in ionischer Form vor. F.N. Marzulli et al., The Journal of Investigative Dermatology 44 , 339-344 (1965), untersuchten die Penetration radioaktiv markierter organischer Phosphorsäureester und Phosphorsäure in vitro durch menschliches Stratum corneum und stellten dabei fest, dass die ionisierte Phosphorsäure nur in ausserordentlich geringem Masse durch die Haut penetrieren kann. Als Schlussfolgerung aus diesen Studien wurde festgehalten, dass neutrale Moleküle relativ leicht die Barriere des Stratum corneum durchdringen, während ionische Formen nur in sehr geringem Masse und mit grossen Schwierigkeiten penetrieren können. Diese Auffassung wird ebenfalls von R.J. Scheuplein, Physiological Reviews 51 , 16-23 (1971), vertreten, der zu dem Schluss gelangt, dass Ionisation die Hautpermeation drastisch reduziert.

Um so überraschender ist die Feststellung, dass pharmazeutisch verwendbare Methandiphosphonsäure-Derivate, in erster Linie solche der nachstehend aufgeführten Formel I, leicht durch die Hautbereiche geschleust werden und somit unmittelbar systemisch wirken können. Diese unerwarteten Erkenntnisse wurden bei in vitro Untersuchungen zur Ermittlung von perkutanen Absorptionseigenschaften nach der Methodik von A.S. Bhatti et al., J. Pharm. Pharmacol., 40 , 45 P (1988) gewonnen. Hierbei wurden entsprechende Diffusionszellen mit einer Zellenoberfläche von 1,27 $cm^2$ und als Membran Schweinehaut als Modell für menschliche Haut verwendet. Die Untersuchungen ergaben, dass signifikante therapeutische Mengen an Wirkstoff durch die Membran diffundiert sind. Diese Ergebnisse konnten bei in-vivo-Studien über die transdermale Absorption von Bisphosphonaten bestätigt werden. Es wurden Experimente an Meerschweinchen mit $^{14}$C-markiertem Wirkstoff durchgeführt. Hierbei wurden 6 mg kalter Bisphosphonatwirkstoff und ungefähr $10^6$ dpm $^{14}$-C-markiertes Bisphosphonat in 200 $\mu$l 2% Klucel in destilliertem Wasser mit einem pH von 7,5 auf eine rasierte Fläche von 3 x 5 cm aufgetragen und diese mit einem Okklusionsverband abgedeckt. Nach 1, 2 und 3 Tagen wurde jeweils das radioaktive Material im ausgeschiedenen Harn gemessen.

Gegenstand der vorliegenden Erfindung sind topisch applizierbare pharmazeutische Präparate enthaltend pharmazeutisch verwendbare Methandiphosphonsäure-Derivate oder deren Salze, ihre Herstellung und Verwendung.

Als pharmazeutisch wirksame Methandiphosphonsäure-Derivate kommen beispielsweise Verbindungen der Formel

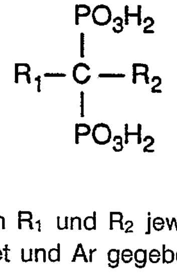

(I)

und ihre Salze in Betracht, worin $R_1$ und $R_2$ jeweils Halogen bedeuten, oder worin $R_1$ Wasserstoff ist und $R_2$ Ar-S- oder $Het_1$-NH- bedeutet und Ar gegebenenfalls substituiertes Phenyl bedeutet und $Het_1$ gegebenenfalls substituiertes, monocyclisches, 5-oder 6-gliedriges Monoaza-, Diaza- oder Thiazaaryl bedeutet, welches über ein Ringkohlenstoffatom gebunden ist, oder worin $R_1$ Wasserstoff oder Hydroxy bedeutet und

$R_2$ für -A-$R_3$ steht, worin A Alkylen bedeutet, und einerseits $R_3$ für $Het_2$ steht, das über ein Ringkohlenstoff- oder Ringstickstoffatom gebundenes $Het_1$ bedeutet, oder andererseits $R_3$ Wasserstoff oder gegebenenfalls durch Alkyl, Cycloalkyl, Ar-Alkyl, Ar-O-Alkyl, Ar-S-Alkyl oder $Het_1$-Alkyl unabhängig voneinander mono- oder disubstituiertes oder durch gegebenenfalls Ar enthaltendes Alkylen disubstituiertes Amino bedeutet, wobei zwei Alkylenkohlenstoffatome zusätzlich über Alkylen miteinander verknüpft sein können und Ar bzw. $Het_1$ jeweils die vorstehend angegebenen Bedeutungen hat, mit der Massgabe, dass, wenn $R_2$ für -A-$R_3$ steht, A Ethylen bedeutet und $R_3$ gegebenenfalls durch $C_1$-$C_3$-Alkyl substituiertes Amino bedeutet, $R_1$ von Hydroxy verschieden ist.

Salze von Verbindungen der Formel I sind insbesondere deren Salze mit Basen, wie von Gruppen Ia, Ib, IIa und IIb des Periodensystems der Elemente abgeleitete Metallsalze, z.B. Alkalimetall-, insbesondere Natrium- oder Kaliumsalze, Erdalkalimetall-, insbesondere Calcium- oder Magnesiumsalze, ebenso Ammoniumsalze mit Ammoniak oder organischen Aminen.

Substituiertes Phenyl ist ein- oder mehrfach, z.B. zwei-, ferner dreifach, substituiert, z.B. durch Niederalkyl, Niederalkoxy, Trifluormethyl und/oder insbesondere Halogen.

Monocyclisches, 5- oder 6-gliedriges Monoaza-, Diaza- oder Thiazaaryl bedeutet beispielsweise Pyrrolyl, Imidazolyl, wie 1-, 2- oder 4-Imidazolyl, Pyrazolyl, wie 1- oder 3-Pyrazolyl, Thiazolyl, wie 2- oder 4-Thiazolyl, Pyridyl, wie 2-, 3- oder 4-Pyridyl. Entsprechende Reste können z.B. durch Alkyl ein- oder mehrfach, z.B. zwei-, ferner dreifach, substituiert sein. Bevorzugte substituierte Reste sind beispielsweise Imidazol-1-yl und Imidazol-5-yl, 5-Niederalkyl-, wie 5-Methyl-thiazol-2-yl, 5-Ethylthiazol-2-yl und 5-n-Butylthiazol-2-yl, sowie 2- und 3-Pyridyl.

Alkyl ist insbesondere Niederalkyl und Alkylen insbesondere Niederalkylen, während Ar-Alkyl beispielsweise gegebenenfalls im Phenylteil wie vorstehend angegeben substituiertes Phenylniederalkyl bedeutet.

Cycloalkyl ist insbesondere $C_3$-$C_7$-Cycloalkyl, wie Cyclopropyl, -butyl, -pentyl, -hexyl oder -heptyl.

Durch Alkyl, Cycloalkyl, Ar-Alkyl, Ar-O-Alkyl, Ar-S-Alkyl oder $Het_1$-Alkyl mono- oder disubstituiertes Amino bedeutet insbesondere Niederalkyl-, $C_3$-$C_7$-Cycloalkyl-, Phenylniederalkyl-, Diniederalkyl-, Niederalkyl-phenylniederalkyl-, Diphenylniederalkylamino, Phenoxyniederalkylamino, Niederalkylphenoxyniederalkyl-amino, Phenoxyniederalkyl-phenylniederalkyl-amino, Diphenoxyniederalkyl-amino, Phenylthioniederalkyl-, Niederalkyl-phenylthioniederalkyl-, Phenylthioniederalkyl-phenylniederalkyl-, Diphenylthioniederalkyl-amino, Niederalkyl-pyridylniederalkyl-amino, Phenylniederalkyl-pyridylniederalkylamino, Phenoxyniederalkyl-pyridylniederalkylamino, Phenylthioniederalkyl-pyridylniederalkyl-amino oder Dipyridylniederalkylamino, wobei der Phenyl- bzw. Pyridylteil gegebenenfalls wie vorstehend angegeben substituiert sein kann.

Durch gegebenenfalls Ar enthaltendes Alkylen, wie 1,4-Butylen oder 1,5-Pentylen, disubstituiertes Amino bedeutet insbesondere Niederalkylenamino oder einen gegebenenfalls wie vorstehend angegeben substituierten Phenylrest enthaltendes Niederalkylenamino, wie Pyrrolidin-1-yl, 2-(4-Chlorphenyl)- 1,4-butylen- oder 3-Phenyl-1,5-pentylen-amino.

Durch Alkylen disubstituiertes Amino, worin zwei Alkylenkohlenstoffatome zusätzlich über Alkylen miteinander verknüpft sind, bedeutet insbesondere Niederalkylenamino, worin zwei, insbesondere nicht benachbarte, Niederalkylenkohlenstoffatome über Niederalkylen, in erster Linie Methylen, miteinander verknüpft sind. Bevorzugt sind z.B. entsprechende 3-Azabicyclo-$C_6$-$C_{10}$-alk-3-yl-Reste.

Die vor- und nachstehend verwendeten Allgemeindefinitionen haben, sofern nicht abweichend definiert, in erster Linie die folgenden Bedeutungen, wobei mit "nieder" bezeichnete Reste oder Verbindungen insbesondere bis und mit 7 C-Atome aufweisen:

Halogen ist insbesondere Halogen mit Atomnummer bis und mit 35, wie Fluor oder Brom, ferner Jod, in erster Linie Chlor.

Niederalkyl ist z.B. Methyl, Ethyl, n-Propyl, Isopropyl, n-Butyl, Isobutyl, sek- und tert-Butyl und umfasst ferner entsprechende Pentyl-, Hexyl- und Heptylreste. Bevorzugt ist $C_1$-$C_4$-Alkyl.

Niederalkoxy ist z.B. Methoxy, Ethoxy, n-Propyloxy, Isopropyloxy und n-Butyloxy und umfasst ferner entsprechende Pentyloxy-, Hexyloxy- und Heptyloxyreste. Bevorzugt ist $C_1$-$C_4$-Alkoxy.

Niederalkylen ist geradkettig oder verzweigt und bedeutet z.B. $C_1$-$C_7$-Alkylen, wie Methylen, Ethylen, Propylen, Butylen, Pentylen, ferner Hexylen und Heptylen, sowie 2-Methyl-1,3-propylen, 2,4- oder 1,5-Dimethyl-1,5-pentylen. Niederalkylen als Substituent von disubstituiertem Amino $R_3$ weist mindestens zwei C-Atome, vorzugsweise 4-6 C-Atome, auf. Niederalkylen, welches zwei Niederalkylenkohlenstoffatome von durch Niederalkylen disubstituiertem Amino miteinander verknüpft, weist insbesondere bis und mit 5 C-Atome auf und bedeutet bevorzugt Methylen.

Die Verbindungen der Formel I und ihre Salze sind bekannt oder können in an sich bekannter Weise hergestellt werden.

So sind beispielsweise Verbindungen der Formel I, worin $R_1$ Wasserstoff ist und $R_2$ Ar-S-bedeutet,

durch Umsetzung von Tetraniederalkylmethandiphosphonat in Gegenwart einer starken Metallbase, wie NaH, mit einem Disulfid der Formel Ar-S-S-Ar und sich anschliessender saurer Hydrolyse des Tetraniederalkylesters zugänglich (s. z.B. EP 100,718).

Entsprechende Verbindungen der Formel I, worin $R_1$ Wasserstoff ist und $R_2$ $Het_1$-NH-bedeutet, kann man z.B. herstellen, indem man ein Gemisch aus $H_3PO_3$ und $PHal_3$, worin Hal Halogen, insbesondere Chlor, bedeutet, mit einem Formylamin der Formel $Het_1$-NH-CHO umsetzt oder ein Amin $Het_1$-$NH_2$ mit einem Orthoameisensäureniederalkylester sowie einem Diniederalkylphosphit erhitzt und das Reaktionsprodukt, z.B. in Gegenwart einer Säure, hydrolysiert (s. z.B. EP 274,346).

Zur Herstellung von Verbindungen der Formel I, worin $R_1$ Wasserstoff ist und $R_2$ für -A-$R_3$ steht und A Alkylen bedeutet, kann man beispielsweise von Verbindungen der Formel $R_3$-A-Hal ausgehen und diese mit einem Tetraniederalkylmethandiphosphonat in Gegenwart einer starken Base, z.B. NaH, umsetzen und den so erhältlichen Tetraniederalkylester von entsprechenden Verbindungen der Formel I z.B. in Gegenwart einer Säure, wie Chlorwasserstoffsäure, hydrolysieren (s. z.B. EP 275,821).

Verbindungen der Formel I, worin $R_1$ Hydroxy bedeutet und $R_2$ für -A-$R_3$ steht, kann man beispielsweise herstellen, indem man eine Carbonsäure der Formel $R_2$-COOH mit einem Phosphorylierungsmittel, wie einem Gemisch aus $H_3PO_3$ und $PHal_3$, umsetzt und unter hydrolytischen Bedingungen aufarbeitet (s. z.B. EP 170,228 und EP 252,505).

Die Methandiphosphonsäure-Derivate der Formel I weisen wertvolle pharmakologische Eigenschaften auf. Insbesondere besitzen sie eine ausgeprägte regulierende Wirkung auf den Calcium-Stoffwechsel von Warmblütern. Ebenso bewirken sie an der Ratte eine ausgeprägte Hemmung der Knochenresorption, die sich sowohl in der Versuchsanordnung gemäss Acta Endocrinol. 78 , 613-24 (1975) als auch im TPTX (Thyroparathyroidectomised) - Rattenmodell anhand der durch Vitamin $D_3$ ausgelösten experimentellen Hyperkalzämie zeigen lässt. Ebenso wird die durch Walker-256-Tumore induzierte Tumorhyperkalzämie nach peroraler Verabreichung gehemmt. Ferner zeigen sie in der Adjuvansarthritis der Ratte in der Versuchsanordnung nach Newbould, Brit. J. Pharmacology 21 , 127(1963) sowie nach Kaibara et al., J. Exp. Med. 159 , 1388-96 (1984) eine deutliche Hemmung auf das Fortschreiten chronisch-arthritischer Prozesse. Sie sind deshalb vorzüglich geeignet als Arzneimittelwirkstoffe für die Behandlung von Erkrankungen, die mit Störungen des Calciumstoffwechsels in Verbindung gebracht werden können, beispielsweise entzündlicher Prozesse in Gelenken, degenerativer Prozesse im Gelenkknorpel, von Osteoporosis, Periodontitis, Hyperparathyreoidismus und von Calciumablagerungen in Blutgefässen oder an prothetischen Implantaten. Günstig beeinflusst werden sowohl Erkrankungen, bei denen eine anomale Ablagerung schwerlöslicher Calciumsalze festzustellen ist, wie solcher aus den Formenkreisen der Arthritis, z.B. Morbus Bechterew, Neuritis, Bursitis, Periodontitis und Tendinitis, Fibrodysplasie, Osteoarthrose oder Artereosklerose, als auch solche, bei denen eine anomale Auflösung harter Körpergewebe im Vordergrund steht, wie die hereditäre Hypophosphatasie, degenerative Prozesse im Gelenkknorpel, Osteoporosen verschiedener Genese, Morbus Paget und die Osteodystrophia fibrosa, ebenso durch Tumore ausgelöste osteolytische Prozesse sowie Hypercalcämie. Einzelne Vertreter der vorstehend definierten Substanzklasse werden bereits therapeutisch eingesetzt.

Die Erfindung betrifft insbesondere topisch applizierbare pharmazeutische Präparate, welche eine pharmazeutisch wirksame Methandiphosphonsäure der Formel I oder ein Salz davon enthalten, worin $R_1$ und $R_2$ jeweils Halogen bedeuten, oder worin $R_1$ Wasserstoff ist und $R_2$ Ar-S- oder $Het_1$-NH- bedeutet und Ar gegebenenfalls substituiertes Phenyl bedeutet und $Het_1$ gegebenenfalls substituiertes, monocyclisches, 5- oder 6-gliedriges Monoaza-, Diaza- oder Thiazaaryl bedeutet, welches über ein Ringkohlenstoffatom gebunden ist, oder worin $R_1$ Wasserstoff oder Hydroxy bedeutet und $R_2$ für -A-$R_3$ steht, worin A Alkylen bedeutet, und einerseits $R_3$ für $Het_2$ steht, das über ein Ringkohlenstoff- oder Ringstickstoffatom gebundenes $Het_1$ bedeutet, oder andererseits $R_3$ Wasserstoff oder gegebenenfalls durch Alkyl, Ar-Alkyl, Ar-O-Alkyl oder $Het_1$-Alkyl unabhängig voneinander mono- oder disubstituiertes oder durch gegebenenfalls Ar enthaltendes Alkylen disubstituiertes Amino bedeutet, wobei zwei Alkylenkohlenstoffatome zusätzlich über Alkylen miteinander verknüpft sein können und Ar bzw. $Het_1$ jeweils die vorstehend angegebenen Bedeutungen hat, mit der Massgabe, dass, wenn $R_2$ für -A-$R_3$ steht, A Ethylen bedeutet und $R_3$ gegebenenfalls durch $C_1$-$C_3$-Alkyl substituiertes Amino bedeutet, $R_1$ von Hydroxy verschieden ist.

Die Erfindung betrifft insbesondere topisch applizierbare pharmazeutische Präparate, welche eine pharmazeutisch wirksame Methandiphosphonsäure der Formel I oder ein Salz davon enthalten, worin $R_1$ und $R_2$ jeweils Halogen bedeuten, oder worin $R_1$ Wasserstoff ist und $R_2$ Ar-S- oder $Het_1$-NH- bedeutet und Ar gegebenenfalls durch Niederalkyl, Niederalkoxy, Trifluormethyl und/oder insbesondere Halogen substituiertes Phenyl bedeutet, und $Het_1$ unsubstituiertes oder durch Niederalkyl substituiertes Thiazolyl bedeutet, oder worin $R_1$ Wasserstoff oder Hydroxy bedeutet und $R_2$ für -A-$R_3$ steht, worin A Niederalkylen bedeutet und einerseits $R_3$ gegebenenfalls durch Niederalkyl substituiertes Imidazolyl, welches über ein

Ringkohlenstoff- oder ein Ringstickstoffatom gebunden ist, oder Pyridyl bedeutet, oder andererseits $R_3$ Wasserstoff oder gegebenenfalls durch Niederalkyl, $C_3$-$C_7$-Cycloalkyl, Ar-Niederalkyl, Ar-O-Niederalkyl, Ar-S-Niederalkyl oder Pyridylniederalkyl unabhängig voneinander mono- oder disubstituiertes oder durch gegebenenfalls Ar enthaltendes Niederalkylen oder durch Niederalkylen, worin zwei nicht benachbarte Niederalkylenkohlenstoffatome zusätzlich über Niederalkylen miteinander verknüpft sind, disubstituiertes Amino bedeutet, und Ar jeweils die vorstehend angegebenen Bedeutungen hat, mit der Massgabe, dass, wenn $R_2$ für -A-$R_3$ steht, A Ethylen bedeutet und $R_3$ gegebenenfalls durch $C_1$-$C_3$-Alkyl substituiertes Amino bedeutet, $R_1$ von Hydroxy verschieden ist.

Die Erfindung betrifft bevorzugt topisch applizierbare pharmazeutische Präparate, welche eine pharmazeutisch wirksame Methandiphosphonsäure der Formel I oder ein Salz, insbesondere Alkalimetall-, z.B. Natriumsalz, davon enthalten, worin $R_1$ und $R_2$ jeweils Halogen, insbesondere Chlor, bedeuten, oder worin $R_1$ Wasserstoff ist und $R_2$ gegebenenfalls durch Halogen, wie Chlor, substituiertes Phenylthio, wie 4-Chlorphenylthio, oder gegebenenfalls durch Niederalkyl, insbesondere $C_1$-$C_4$-Alkyl, wie Methyl, substituiertes Thiazolylamino, wie Thiazol-2-yl-, 5-n-Butylthiazol-2-yl-, 5-Ethylthiazol-2-yl- oder 5-Methylthiazol-2-ylamino, bedeutet, oder worin $R_1$ Wasserstoff oder Hydroxy bedeutet und $R_2$ für -A-$R_3$ steht, worin A $C_1$-$C_7$-Alkylen, wie Methylen, Ethylen, Propylen oder Pentylen, bedeutet und $R_3$ ein über ein Ringkohlenstoff- oder Ringstickstoffatom gebundenes, gegebenenfalls durch Niederalkyl, insbesondere $C_1$-$C_4$-Alkyl, wie Methyl, substituiertes Imidazolyl, wie Imidazol-1-yl, Imidazol-5-yl, 1-Methylimidazol-2-yl oder 4-Methyl-imidazol-5-yl, oder Pyridyl, wie 2- oder 3-Pyridyl, bedeutet, oder worin $R_1$ Hydroxy ist und $R_2$ für -A-$R_3$ steht, worin A die oben angegebene Bedeutung hat und $R_3$ Amino, Di-$C_1$-$C_5$-alkylamino, wie Dimethyl-, N-Methyl-N-n-propylamino oder N-Methyl-N-n-pentylamino, N-$C_3$-$C_7$-Cycloalkylamino, wie N-Cycloheptylamino, N-$C_1$-$C_4$-Alkyl-N-phenyl-$C_1$-$C_5$-alkylamino, wie N-Methyl-N-(2-phenylethyl)-amino, N-Methyl-N-(3-phenylpropyl)-amino oder N-Methyl-N-(5-phenylpentyl)-amino, N-$C_1$-$C_4$-Alkyl-N-phenoxy-$C_1$-$C_4$-alkyl, wie N-Methyl-N-(3-phenoxypropyl)-amino, N-$C_1$-$C_4$-Alkyl-N-phenylthio-$C_1$-$C_4$-alkyl, wie N-Methyl-N-(2-phenylthioethyl)-amino, N-$C_1$-$C_4$-Alkyl-N-pyridyl-$C_1$-$C_4$-alkylamino, wie N-Methyl-N-[3-(2-pyridyl)-propyl]-amino, $C_4$-$C_6$-Alkylenamino, welches gegebenenfalls durch gegebenenfalls Halogen, wie Chlor, enthaltendes Phenyl, wie 4-Chlorphenyl, substituiert ist, z.B. Piperidin-1-yl, welches gegebenenfalls, insbesondere in Position 4, durch Phenyl substituiert ist, oder Pyrrolidin-1-yl, welches gegebenenfalls, insbesondere in Position 3, durch 4-Chlorphenyl substituiert ist, oder 1,5-Di-$C_1$-$C_4$-alkyl-, wie 1,5-Dimethyl-3-aza-bicyclo[3.1.1]hept-3-yl bedeutet, oder worin $R_1$ Hydroxy ist und $R_2$ für -A-$R_3$ steht, worin A $C_1$-$C_4$-Alkylen, insbesondere Methylen, bedeutet und $R_3$ Wasserstoff ist, mit der Massgabe, dass, wenn $R_2$ für -A-$R_3$ steht, A Ethylen bedeutet und $R_3$ gegebenenfalls durch $C_1$-$C_3$-Alkyl substituiertes Amino bedeutet, $R_1$ von Hydroxy verschieden ist.

Die Erfindung betrifft in erster Linie topisch applizierbare pharmazeutische Präparate, welche eine pharmazeutisch wirksame Methandiphosphonsäure der Formel I oder ein Salz, insbesondere Alkalimetall-, z.B. Natriumsalz, davon enthalten, worin $R_1$ Hydroxy ist und $R_2$ für -A-$R_3$ steht, worin A $C_2$-$C_7$-Alkylen, wie Ethylen, Propylen oder Pentylen, bedeutet und $R_3$ Amino, Di-$C_1$-$C_4$-alkylamino, wie Dimethyl- oder N-Methyl-N-n-propylamino, N-$C_3$-$C_7$-Cycloalkylamino, wie N-Cycloheptylamino, N-$C_1$-$C_4$-Alkyl-N-phenyl-$C_1$-$C_4$-alkylamino, wie N-Methyl-N-(3-phenylpropyl)-amino, N-$C_1$-$C_4$-Alkyl-N-phenoxy-$C_1$-$C_4$-alkylamino, wie N-Methyl-N-(3-phenoxypropyl)-amino, oder N-$C_1$-$C_4$-Alkyl-N-phenylthio-$C_1$-$C_4$-alkylamino, wie N-Methyl-N-(2-phenylthioethyl)-amino, bedeutet, mit der Massgabe, dass, wenn $R_2$ für -A-$R_3$ steht, A Ethylen bedeutet und $R_3$ gegebenenfalls durch $C_1$-$C_3$-Alkyl substituiertes Amino bedeutet, $R_1$ von Hydroxy verschieden ist.

Die Erfindung betrifft in erster Linie topisch applizierbare pharmazeutische Präparate, welche eine pharmazeutisch wirksame Methandiphosphonsäure der Formel I oder ein Salz davon, insbesondere Alkalimetall-, z.B. Natriumsalz, davon enthalten, worin $R_1$ Hydroxy ist und $R_2$ für -A-$R_3$ steht, worin A $C_1$-$C_7$-Alkylen, insbesondere $C_1$-$C_4$-Alkylen, wie Methylen, bedeutet und $R_3$ ein über ein Ringkohlenstoff- oder Ringstickstoffatom gebundenes, gegebenenfalls durch $C_1$-$C_4$-Alkyl, wie Methyl, substituiertes Imidazolyl, wie insbesondere Imidazol-1-yl, ferner Imidazol-5-yl, 1-Methyl-imidazol-2-yl oder 4-Methyl-imidazol-5-yl, bedeutet.

Die Erfindung betrifft in erster Linie topisch applizierbare pharmazeutische Präparate, welche eine pharmazeutisch wirksame Methandiphosphonsäure ausgewählt aus folgenden Verbindungen der Formel I:

4-Amino-1-hydroxy-butan-1,1-diphosphonsäure, 6-Amino-1-hydroxy-hexan-1,1-diphosphonsäure, 3-(N-Methyl-N-n-pentyl-amino)-1-hydroxy-propan-1,1-diphosphonsäure, 3-[N-(3-Phenylpropyl)-N-methyl-amino]-1-hydroxy-propan-1,1-diphosphonsäure, 3-(N-Methyl-N-5-phenylpentyl-amino)-1-hydroxypropan-1,1-diphosphonsäure, 3-[N-Methyl-N-3-(2-pyridyl)-propyl-amino]-1-hydroxy-propan-1,1-diphosphonsäur e, 1-Hydroxy-3-[N-methyl-N-(3-phenoxypropyl)-amino]-propan-1,1-diphosphonsäure, 1-Hydroxy-3-[N-methyl-N-(2-phenoxyethyl)-amino]-propan-1,1-diphosphonsäure, 4-(4-Phenyl-piperidin-1-yl)-1-hydroxy-butan-1,1-diphosphonsäure, 1-Hydroxy-3-(1-piperidino)-propan-1,1-diphosphonsäure, 1-Hydroxy-3-[3-(4-chlorphenyl)-pyrrolidin- 1 -yl]-propan-1,1-diphosphonsäure, 1-Hydroxy-2-(imidazol-1-yl)-ethan-1,1-diphosphonsäure, 2-(1-Methyl-imidazol-2-yl)-ethan-1,1-diphosphonsäure,1-Hydroxy-2-(4-methyl-imidazol-5-yl)-ethan-1,1-diphosphonsäure,1-Hydroxy-2-(imidazol-5-yl)-ethan-1,1-diphosphonsäure,1-Hydroxy-2-(3-pyridyl)-ethan-1,1-diphosphonsäure,2-(2-Pyridyl)-ethan-1,1-diphosphonsäure, 1-[(5-nButyl-2-thiazolyl)-amino]-methan-1,1-diphosphonsäure,1-[(5-Methyl-2-thiazolyl)-amino]-methan-1,1-diphosphonsäure, 1-[(2-Thiazolyl)-amino]-methan-1,1-diphosphonsäure,1-Hydroxy-ethan-1,1-diphosphonsäure,1-(4-Chlor-phenylthio)-methan-1,1-diphosphonsäure,1,1-Dichlormethan-1,1-diphosphonsäure oder 3-(1,5-Dimethyl-3-azabicyclo[3.1.1]hept-3-yl)-1-hydroxy-propan-1,1-diphosphonsäure, ebenso 1-[(5-Ethyl-2-thiazolyl)-amino]-methan-1,1-diphosphonsäure,3-[N-(2-Phenylethyl)-N-methyl-amino]-1-hydroxy-propan-1,1-diphosphonsäure,3-[N-(2-Phenylthioethyl)- N-methyl-amino]-1-hydroxy-propan-1,1-diphosphonsäure, 1-Hydroxy-3-(pyrrolidin-1-yl)-propan-1,1-diphosphonsäure oder 2-(N-Cycloheptylamino)-ethan-1,1-diphosphonsäure oder ein pharmazeutisch verwendbares Salz davon, enthalten.

Gegenstand der Erfindung ist ebenso ein Verfahren zur Herstellung der erfindungsgemässen topisch applizierbaren pharmazeutischen Präparate, welches nach an sich bekannter Methodik erfolgen kann und dadurch gekennzeichnet ist, dass man ein pharmazeutisch verwendbares Methandiphosphonat-Derivat mit üblichen pharmazeutisch verwendbaren Hilfs- und Zusatzstoffen verarbeitet.

Ein weiterer Gegenstand der Erfindung ist ein Verfahren zur Steigerung der Resorption von pharmazeutisch verwendbaren Methandiphosphonsäure-Derivaten, insbesondere von Verbindungen der Formel I, welches dadurch gekennzeichnet ist, dass man eine Verbindung der Formel I oder ein Salz davon in eine pharmazeutische Zusammensetzung zur topischen Applikation einarbeitet.

Die Erfindung betrifft dabei namentlich die in den Beispielen beschriebenen pharmazeutischen Präparate und Verfahren zu deren Herstellung.

Die Verbindungen der Formel I können je nach Wahl der Ausgangsstoffe und Arbeitsweisen in Form eines möglichen Isomeren oder eines Gemisches derselben vorliegen, beispielsweise als optische Isomere, wie Enantiomere oder Diastereomere, oder geometrische Isomere, wie cis-trans-Isomere. Dabei liegen die optischen Isomeren in Form der reinen Antipoden und/oder als Racemate vor.

Die Verbindungen der Formel I können auch in Form ihrer Hydrate eingesetzt werden oder andere zur Kristallisation verwendete Lösungsmittel einschliessen.

Die erfindungsgemässen topisch anwendbaren pharmazeutischen Präparate enthalten die pharmazeutisch verwendbaren Verbindungen der Formel I beispielsweise in einer pharmakologisch wirksamen Menge, zusammen mit einem pharmazeutisch anwendbaren Zusatz-oder Hilfsmaterial. Die tägliche Dosierung des Wirkstoffs hängt von dem Alter und dem individuellen Zustand, sowie von der Applikationsweise ab.

Als topisch anwendbare pharmazeutische Präparate kommen in erster Linie Cremen, Salben und Gele, ferner Pasten, Schäume, Tinkturen und Lösungen in Frage, die von etwa 0,5 bis etwa 5 % des Wirkstoffes enthalten.

Cremen oder Lotionen sind Oel-in-Wasser Emulsionen, die mehr als 50 % Wasser aufweisen. Als ölige Grundlage verwendet man in erster Linie Fettalkohole, insbesondere solche mit 12 bis 18 C-Atomen, z.B. Lauryl-, Cetyl- oder Stearylalkohol, Fettsäuren, insbesondere solche mit 10 bis 18 C-Atomen, z.B. Palmitin- oder Stearinsäure, flüssige bis feste Wachse, z.B. Isopropylmyristat, Wollwachs oder Bienenwachs, und/oder Kohlenwasserstoffe, insbesondere füssige, halbfeste oder feste Stoffe oder Gemische derselben, z.B. Vaselin (Petrolatum) oder Paraffinöl. Als Emulgatoren kommen oberflächenaktive Substanzen mit vorwiegend hydrophilen Eigenschalten in Frage, wie entsprechende nichtionische Emulgatoren, z.B. Fettsäureester von Polyalkoholen oder Ethylenoxidaddukte davon, insbesondere entsprechende Fettsäureester mit (Poly-)Ethylenglykol, (Poly-)Propylenglykol oder Sorbit, wobei der Fettsäureanteil insbesondere 10 bis 18 C-Atome aufweist, in erster Linie partielle Glycerinfettsäurester oder partiellle Fettsäureester der Polyhydroxyethylensorbitans, wie Polyglycerinfettsäureester oder Polyoxyethylensorbitanfettsäureester (Tweens), ferner Polyoxyethylenfettalkoholether oder -fettsäureester, wobei der Fettalkoholanteil insbesondere 12 bis 18 C-Atome und der Fettäureanteil insbesondere 10 bis 18 C-Atome aufweist,insbesondere solche mit etwa 2 bis 23 Ethylenglycol- bzw. Ethylenoxideinheiten, wie Polyhydroxyethylencetylstearylether (z.B. Cetomacrogol), Polyhydroxyethylen(4)-laurylether sowie Polyhydroxyethylen-glycerin-fettsäureester (z.B. Tagat S), oder entsprechende ionische Emulgatoren, wie Alkalimetallsalze von Fettalkoholsulfaten, insbesondere mit 12 bis 18 C-Atomen im Fettalkoholanteil, z.B. Natriumlaurylsulfat, Natriumcetylsulfat oder Natriumstearylsulfat, die man üblicherweise in Gegenwart von Fettalkoholen, z.B. Cetylalkohol oder Stearylalkohol, verwendet. Zusätze zur Wasserphase sind u.a. Mittel, welche die Austrocknung der Cremen verhindern, z.B. Feuchthaltemittel, wie Polyalkohole, wie Glycerin, Sorbit, Propylenglykol und/oder Polyethylenglykole, ferner Konservierungsmittel, Riechstoffe, etc.

Salben oder Lotionen sind Wasser-in-Oel Emulsionen, die bis zu 70 %, vorzugsweise jedoch von etwa 20 % bis etwa 50 % Wasser oder wässrige Phase enthalten. Als Fettphase kommen in erster Linie Kohlenwasserstoffe, z.B. Vaseline, Paraffinöl und/oder Hartparaffine in Frage, die zur Verbesserung des

Wasserbindungsvermögens vorzugsweise geeignete Hydroxyverbindungen, wie Fettalkohole oder Ester davon, z.B. Cetylalkohol oder Wollwachsalkohole bzw. Wollwachs, enthalten. Emulgatoren sind entsprechende lipophile Substanzen, beispielsweise der vorstehend aufgeführten Art, wie Sorbitanfettsäureester (Spans), z.B. Sorbitanoleat und/oder Sorbitanisostearat. Zusätze zur Wasserphase sind u.a. Feuchthaltungsmittel, wie Polyalkohole, z.B. Glycerin, Propylenglykol, Sorbit und/oder Polyethylenglykol, sowie Konservierungsmittel, Riechstoffe, etc.

Mikroemulsionen sind isotrope Systeme, deren Grundlage aus folgenden vier Komponenten besteht: Wasser, einem Emulgator, beispielsweise der vorstehend aufgeführten Art, wie einem Tensid, z.B. Emulgin, einem Lipid, wie einem unpolaren Oel, z.B. Paraffinöl, und einem Alkohol mit einer lipophilen Gruppe, z.B. 2-Octyldodecanol. Wenn erwünscht, können die Mikroemulsionen mit anderen Zusatzmitteln versetzt werden.

Fettsalben sind wasserfrei und enthalten als Grundlage insbesondere Kohlenwasserstoffe, z.B. Paraffin, Vaseline und/oder flüssige Paraffine, ferner natürliches oder partialsynthetisches Fett, wie Fettsäureester des Gylcerins, z.B. Kokosfettsäuretriglycerid, oder vorzugsweise gehärtete Oele, z.B. hydriertes Erdnuss- oder Rizinusöl, ferner Fettsäurepartialester des Glycerins, z.B. Glycerinmono- und -distearat, sowie z.B. die im Zusammenhang mit den Salben erwähnte, die Wasseraufnahmefähigkeit steigernden Fettalkohole, Emulgatoren und/oder Zusätze.

Bei den Gelen wird zwischen wässrigen, wasserfreien oder wasserarmen Gelen unterschieden, die aus quellbaren, gelbildenden Materialien bestehen. In erster Linie kommen transparente Hydrogele auf der Grundlage anorganischer oder organischer Makromoleküle zum Einsatz. Hochmolekulare anorganische Komponenten mit gelbildenden Eigenschaften sind überwiegend wasserhaltige Silikate, wie Aluminiumsilikate, z.B. Bentonit, Magnesium-Aluminium-Silikate, z.B. Veegum, oder kolloidale Kieselsäure, z.B. Aerosil. Als hochmolekulare organische Stoffe werden beispielsweise natürliche, halbsynthetische oder synthetische Makromoleküle verwendet. Natürliche und halbsynthetische Polymere leiten sich beispielsweise von Polysacchariden mit den unterschiedlichsten Kohlenhydratbausteinen ab, wie Cellulosen, Stärken, Tragant, arabisches Gummi und Agar-Agar, sowie Gelatine, Alginsäure und deren Salze, z.B. Natriumalginat, und deren Derivate, wie Niederalkylcellulosen, z.B. Methyl- oder Ethylcellulosen, Carboxy- oder Hydroxyniederalkylcellulose, z.B. Carboxymethyl- oder Hydroxyethylcellulosen. Die Bausteine synthetischer, gelbildender Makromoleküle sind beispielsweise entsprechend substituiere ungesättigte Aliphate, wie Vinylalkohol, Vinylpyrrolidin, Acryl- oder Methacrylsäure. Als Beispiele für solche Polymere sind Polyvinylalkohol-Derivate, wie Polyviol, Polyvinylpyrrolidine, wie Kollidon, Polyacrylate bzw. Polymethacrylate, insbesondere mit einem Molekulargewicht von etwa 80000 bis etwa 1 Million, oder deren Salze, wie Rohagit S, Eudispert oder Carbopol, zu nennen. Den Gelen können übliche Zusatzmittel, wie Konservierungs- oder Riechstoffe, zugegeben werden.

Schäume werden z.B. aus Druckbehältern verabreicht und sind in Aerosolform vorliegende flüssige Oel-in-Wasser Emulsionen, wobei gegebenenfalls halogenierte Kohlenwasserstoffe, wie Alkane, z.B. Propan oder Butan, oder Chlorfluorniederalkane, z.B. Dichlordifluormethan und Dichlortetrafluorethan, als Treibmittel verwendet werden. Als Oelphase verwendet man u.a. Kohlenwasserstoffe, z.B. Paraffinöl, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyethylen-sorbitanfettsäureester (Tweens), und solchen mit vorwiegend lipophilen Eigenschaften, wie Sorbitanfettsäureester (Spans). Dazu kommen die üblichen Zusätze, wie Konservierungsmittel, etc.

Tinkturen und Lösungen weisen meistens eine äthanolische Grundlage auf, der gegebenenfalls Wasser zugesetzt wird und der u.a. Polyalkohole, z.B. Glycerin, Glykole, und/oder Polyethylenglykol, als Feuchthaltemittel zur Herabsetzung der Verdunstung, und rückfettende Substanzen, wie Fettsäureester mit niedrigen Polyethylenglykolen, d.h. im wässrigen Gemisch lösliche, lipophile Substanzen als Ersatz für die der Haut mit dem Ethanol entzogenen Fettsubstanzen, und, falls notwendig, andere Hilfs-und Zusatzmittel beigegeben sind. Entsprechende Tinkturen oder Lösungen können auch mittels geeigneter Vorrichtungen in Sprayform appliziert werden.

Die Herstellung der topisch verwendbaren pharmazeutischen Präparate erfolgt in an sich bekannter Weise, z.B. durch Lösen oder Suspendieren des Wirkstoffs in der Grundlage oder in einem Teil davon, falls notwendig. Bei Verabreichung des Wirkstoffs als Lösung wird dieser in der Regel vor der Emulgierung in einer der beiden Phasen gelöst; bei Verabreichung als Suspension wird er nach der Emulgierung mit einem Teil der Grundlage vermischt und dann dem Rest der Formulierung beigegeben.

Die vorliegende Erfindung betrifft in erster Linie mehrschichtige therapeutische Systeme für die transdermale Applikation von pharmazeutisch verwendbaren Methandiphosphonsäure-Derivaten, insbesondere solchen der Formel I, und ihrer Salze, welche im wesentlichen folgende Bestandteile enthalten:

(1) eine geschlossene und für die Bestandteile der nachfolgenden Schichten der Wirkstoffzubereitung

undurchlässige Deckfolie,
(2) ein an die Deckfolie (1) einseitig anschliessendes Reservoir für den Wirkstoff, falls dieser nicht in die Kleberschicht eingearbeitet ist,
(3) eine Kleberschicht und
(4) eine abziehbare Schutzfolie.

Das erfindungsgemässe therapeutische System für die transdermale Applikation von Methandiphosphonsäure-Derivaten der Formel I hat vorzugsweise die Form eines Pflasters mit einer Grundfläche, welche mindestens so gross ist wie die für die Applikation vorgesehene Hautfläche und wie sie für festen Sitz über den gesamten Behandlungszeitraum erforderlich ist. Die Grundfläche muss so gross gewählt werden, dass ausreichende Mengen der aktiven Bestandteile der Wirkstoffzubereitung (z.B. Wirkstoff und die perkutane Absorption verbessernde Mittel, im folgenden Penetrationsverstärker) von der Haut aufgenommen werden können. Obwohl sehr grosse Oberflächen der Haut zur Aufnahme des Pflasters theoretisch zur Verfügung stehen, beträgt aus Komfortgründen die angestrebte Grundfläche des Pflasters ca. 200 $cm^2$, in erster Linie ca. 20 bis ca. 30 $cm^2$.

Die geometrische Form des Pflasters ist beliebig, z.B. oval, elliptisch, kreisförmig, rechteckig, gegebenenfalls mit abgerundeten Ecken, oblong oder rechteckig mit ein oder zwei abgerundeten Laschen. Auch andere Formen sind möglich.

Die Deckfolie (1) besteht aus einem Material oder einer Kombination von Materialien, die für die Bestandteile der im Reservoir (2) enthaltenen Formulierung undurchlässig sein müssen. Sie dient als Schutz- und als Trägerschicht. Zur Herstellung der Deckfolie können Hoch- oder Niederdruckpolymere wie Polyethylen, Polypropylen, Polyvinylchlorid, Polyurethan, Polyethylenterephthalat oder auch Celluloseacetat oder Vinylacetat-Vinylchlorid-Copolymere und Kombinationen, insbesondere Verbundfolien daraus, verwendet werden. Bevorzugt ist eine undurchlässige, flexible Deckfolie, welche sich der Ausformung der betreffenden Körperpartie anpasst, worauf das Pflaster angebracht ist, und welche aus entsprechenden, für die Herstellung heiss formgesiegelter Systeme anwendbaren Materialien besteht.

Das Reservoir (2) für den Wirkstoff ist zwischen der Deckfolie (1) und der Kleberschicht (3) und diese wiederum auf der abziehbaren Schutzfolie (4) angeordnet und enthält alle wesentlichen Bestandteile der Wirkstoffzubereitung. Das Reservoir hat die Aufgabe, den Wirkstoff in einem begrenzten Raum gesammelt zur Abgabe an die Haut zu halten. Es kann eine flüssige, halbfeste oder feste Wirkstoffzübereitung enthalten oder als eine homogene oder heterogene Polymermatrix ausgebildet sein, die selbst die Wirkstoffe enthält. In einer bevorzugten Ausführung wird die wirkstoffhaltige Reservoirschicht als Klebermatrix ausgebildet, die in diesem Falle als sogenanntes Monolith-System keiner zusätzlichen Kleberschicht bedarf. In einer anderen bevorzugten Ausführung wird als Reservoir eine flüssige, halbfeste oder bei Hauttemperatur schmelzbare wirkstoffhaltige Zubereitung in einem Faservlies oder einem polymeren Schaumstoff festgehalten.

Das Reservoir kann, falls es z.B. durch eine den Wirkstoff enthaltende feste oder poröse Matrix gebildet werden, beispielsweise direkt der Schutzfolie (4) aufliegen.

Es kann aber auch, falls es nicht selbst eine Klebermatrix darstellt oder in eine Kleberschicht eingearbeitet ist, auf einer Kleberschicht und diese wiederum auf der abziehbaren Folie haften. Weiterhin kann zwischen das Reservoir und einer Kleberschicht, die ihrerseits auf einer abziehbaren Folie (4) aufliegt, eine poröse oder permeable Membran angeordnet sein.

So ist z.B. eine Anordnung, bei welcher das Reservoir in festem Kontakt mit der Haut steht, an sich bekannt und z.B. in der Britischen Patentanmeldung 2,021,950 beschrieben. Dabei ist die Deckfolie (1) flächenmässig grösser als die vom Reservoir (2) eingenommene Fläche, greift also über diese hinaus, wobei der überstehende Teil der Deckfolie (1) mit einer Kleberschicht versehen ist und auf der Haut haftet. Die abziehbare Schutzfolie (4) liegt dabei auf der Kleberschicht (3) und auf dem Reservoir (2), wobei letzteres auch durch eine zusätzliche Membran begrenzt sein kann.

Ein Beispiel für ein System, in dem das Reservoir (2) z.B. in festem Kontakt mit der Kleberschicht (3) steht, wobei die Wirkstoffgrundlage sowohl im Reservoir als auch in der Kleberschicht enthalten sein kann, ist in der U.S. Patentschrift 4,597,961 beschrieben. Die Deckfolie (1) ist ebenfalls flächenmässig grösser als die vom Reservoir (2) eingenommene Fläche und greift über diese hinaus. Die Kleberschicht (3) bedeckt sowohl das Reservoir (2) als auch den überstehenden Teil der Deckfolie (1). Die abziehbare Schutzfolie (4) liegt auf der Kleberschicht.

Die als Gerüststruktur zum Immobilisieren flüssiger, halbfester oder bei Hauttemperatur schmelzender Wirkstoffzubereitungen verwendbaren Träger bestehen beispielsweise aus natürlichen oder synthetischen Polymeren, wie Baumwolle, Cellulose, regenerierte Cellulose, Polyamide, Polyester, Polyurethane oder Cellulose-Derivate, z.B. solche der nachstehend beschriebenen Art, Viscose oder Polypropylen. Insbesondere werden derartige faserartige Strukturen in Vliesform sowie in Form von Geweben, Gewirken, ferner

Schäumen eingesetzt.

Das Reservoir (2) kann auch flüssiges polymeres Material enthalten, worin die Wirkstoffzubereitung oder Bestandteile davon homogen verteilt sind. Solche polymeren Materialien sind z.B. Siliconkautschuk (Silicone), z.B. lineare Organosiloxane, worin jedes Siliciumatom in der Siloxankette durch zwei gleiche oder verschiedene Alkyl-, z.B. Methyl- oder Aethyl-, Aryl-, z.B. Phenyl-, Alkenyl-, z.B. Vinyl-oder Allyl-, Alkylaryl-, z.B. Tolyl- oder Xylyl-, oder Aralkyl-, z.B. Benzylreste, und jedes terminale Siliciumatom durch drei der genannten organischen Reste substituiert ist. Die Herstellung dieser Silicone ist in den U.S. Patentschriften 2,541,137, 2,723,966, 2,863,846,2,890,188, 2,927,907, 3,002,951 und 3,035,016 beschrieben.

Das Reservoir (2) kann ausser dem flüssigen polymeren Material und der Wirkstoffzubereitung noch andere Flüssigkeiten wie Glycerin oder Propylenglycol sowie Wasser enthalten und die in der U.S. Patentschrift 4,291,015 beschriebenen Abgabeeigenschaften haben.

Vorzugsweise besteht der Inhalt des Reservoirs (2) ausschliesslich aus der Wirkstoffzubereitung selbst, die den Penetrationsverstärker, insbesondere Ethanol, den Wirkstoff und gegebenenfalls weitere Hilfsstoffe, z.B. Geliermittel bzw. gegebenenfgalls viskositätserhöhende Hilfsmittel, wie Polyvinylalkohol, Polyvinylpyrrolidon, Cellulosederivate, z.B. solche der aufgeführten Art, und Gelatine, enthält.

Das Reservoir (2) kann ausserdem mit einer permeablen Schicht versehen sein, welche die erforderliche Durchlässigkeit für den Wirkstoff und den Penetrationsverstärker besitzt. Diese Schicht steuert die Abgabegeschwindigkeiten des Penetrationsverstärkers und/oder des Wirkstoffs aus dem System auf die Haut und wird auch als Kontroll- oder Steuermembran bezeichnet.

Die für die erfindungsgemässen therapeutischen Systeme verwendbaren Materialien zur Herstellung der permeablen Schicht sind an sich bekannt. Solche Membranmaterialien können homogen (Diffusionsmembranen), oder makrostrukturiert (poröse Membranen) sein. Letztere können als schwammförmiges Gebilde mit einer löchrigen Gerüststruktur aus polymerem Material aufgefasst werden, worin untereinander verbundene Zwischenräume und Poren dispergiert sind. Membranmaterialien, welche die Abgabegeschwindigkeit steuern, können aus isotropem Material mit homogener Struktur oder aus anisotropem Material mit nicht-homogener Struktur bestehen. Solche Materialien sind kommerziell erhältlich und können auf verschiedene Weise hergestellt werden, z.B. wie von R.E. Kesting, Synthetic Polymer Membranes, McGraw Hill, Chapters 4 und 5, 1971, J.D. Ferry, Ultrafiltration Membranes, Chemical Review, Vol. 18, Seite 373, 1984 beschrieben.

Membranmaterialien mit 5 bis 95 Vol% Hohlräumen und einer effektiven Porengrösse von ca. 1,0 x $10^{-9}$ m bis 1,0 x $10^{-4}$ m sind besonders geeignet. Vor allem eignen sich Membranmaterialien mit Porengrössen kleiner als ca. 5,0 x $10^{-9}$ m und molekularer Diffusion. Für optimale Resultate wird auf den Stand der Technik und die bekannten Ausführungsformen mit bekannten Membranmaterialien und bekannter Formgebung verwiesen, welche eine optimale Abgabegeschwindigkeit des Wirkstoffs gewährleisten. Insbesondere muss das Membranmaterial gegenüber dem Wirkstoff und dem verwendeten Penetrationsverstärker chemisch resistent sein.

Im folgenden ist eine Aufzählung von geeigneten Membranmaterialien angegeben, welche als nicht erschöpfend aufzufassen ist:

- Polycarbonate, z.B. lineare Polyester von Kohlensäurederivaten, welche Carbonatgruppen in der Polymerkette enthalten, und z.B. durch Umsetzung von Dihydroxyaromaten mit Phosgen herstellbar sind. Solche Materialien sind unter dem Warenzeichen Lexan® von General Electric erhältlich;
- Polyvinylchloride, z.B. das PVC, welches unter dem Warenzeichen Geon® 121 der Firma Goodrich erhältlich ist;
- Polyamide vom Typ Polyhexamethylenadipamid oder solche Polyamide, welche unter dem generischen Namen "Nylon" bekannt sind. Ein besonders geeignetes Material wird unter dem Warenzeichen Nomex® von DuPont verkauft;
- Acrylsäurecopolymere, z.B. solche, welche unter den Handelsnamen Dynel® verkauft werden und zu ca. 60 % aus Polyvinylchlorid und zu 40 % aus Acrylonitril bestehen, sowie Styrol-Acrylsäurecopolymere und ähnliche;
- Polysulfone mit Diphenylsulfon-Gruppen in der linearen Kette. Solche Polymere werden von Union Carbide unter der Bezeichnung P-1700 verkauft;
- Halogenierte Polymere, wie Polyvinylidenfluoride, welche z.B. unter dem Warenzeichen Kynar® von Pennwalt vertrieben werden; Polyvinylfluoride, welche unter dem Warenzeichen Tedlar® bei DuPont erhältlich sind, sowie Polyfluorohalocarbon erhältlich unter dem Warenzeichen Aclar® bei Allied Chemical;
- Polychlorether, welche unter dem Warenzeichen Penton® von Hercules vertrieben werden, sowie andere ähnliche thermoplastische Polymere;
- Acetalpolymere wie die Polyformaldehyd-Polymere, welche von DuPont unter dem Warenzeichen Delrin® vertrieben werden u.ä.

- Acrylsäureresinate, wie Polymethylmethacrylat, Poly-n-butyl-methacrylat u.ä.
- Polyethylen und Copolymere des Ethylens z.B. mit Vinylacetat oder Acrylaten.
- Andere Polymere, wie Polyurethane, Polyimide, Polybenzimidazole, Polyvinylacetat, aromatische und aliphatische Polyether, Celluloseester, z.B. Cellulosetriacetat, Cellulose, Colledion® (Cellulosenitrat mit 11 % Stickstoff), Epoxyresinate, Polyolefine, z.B. Polyethylen-polypropylen, poröses Gummi, Polyvinylpolypyrrolidon, quervernetzter Polyvinylalkohol, Copolymerisate aus Vinylpyrrolidon und Vinylalkoholen, Polyelektrolytstrukturen, welche aus zwei ionisch assoziierten Polymeren bestehen, wie in den U.S. Patentschriften 3,549,016 und 3,546,142 beschrieben, Polystyrolderivate wie Polystyrolnatriumsulfonate oder Polyvinylbenzyltrimethylammoniumchloride, Polyhydroxyethylmethylacrylate, Polyisobutylvinylether und ähnliche Polymere können ebenfalls verwendet werden. Weitere Copolymere, welche durch Copolymerisation von verschiedenen Mengen der den genannten Polymeren zugrundeliegenden Monomeren erhältlich sind, sind ebenfalls zur Herstellung des die Abgabegeschwindigkeit Wirkstoffs und/oder des Penetrationsverstärkers bestimmenden Membranmaterials verwendbar.

Bei Verwendung einer permeablen Membran sind mehrere Anordnungen möglich: Die Wirkstoffzubereitung befindet sich zwischen der Deckfolie (1) und der Membranschicht. In dieser Anordnung wird von der Deckfolie und der Membran ein Volumen gebildet, welches gegebenenfalls durch mehrere Kammern unterteilt sein kann. Die Deckfolie (1) und die Membran sind bei bestimmten Ausführungsformen am Rand selbst miteinander verbunden, z.B. verschweisst oder verklebt. In diesen Ausführungsformen sind der Wirkstoff und der Penetrationsverstärker im selben Reservoir enthalten. Diese Ausführungsformen sind bevorzugt, wenn die Wirkstoffzubereitung flüssig oder halbfest ist.

Man kann auch gemäss der in der Deutschen Offenlegungsschrift 3,205,258 beschriebenen Ausführungsform das von der Deckfolie (1) und der Membran gebildete Volumen nur mit dem Penetrationsverstärker, z.B. Ethanol, und gegebenenfalls mit einem Geliermittel bzw. viskositätserhöhende Hilfsmittel, wie Gelatine füllen und auf die andere Seite der Membran die Wirkstoffzubereitung auftragen. Die Membran würde hierbei nur die Diffusionsgeschwindigkeit des Penetrationsverstärkers steuern. Der Wirkstoff kann in einer getrennten Schicht zwischen Membran und Kleberschicht (3) und gegebenenfalls oder ausschliesslich in der Kleberschicht (3) vorhanden sein.

Das Reservoir (2) kann ausserdem in mehrere Kammern unterteilt sein. Diese Unterteilung eignet sich für flüssige Wirkstoffzubereitungen und verhindert, dass diese bei Bildung von Hohlräumen oder Falten infolge unebener Lagerung des Pflasters absinken und sich am tiefsten Punkt des Systems anreichern. Besonders vorteilhaft ist eine Unterteilung, wenn die Reservoirschicht eine Fläche von mehr als 30 $cm^2$ einnimmt. Die Einteilung der Kammern ist beliebig. So sind z.B. die radiale Anordnung der Stege vom Mittelpunkt des Pflasters, vertikale, horizontale Abgrenzungen, schräge Linien etc. möglich.

Die Unterteilung der Kammern, insbesondere durch Stege oder Siegelnähte, kann durch Verschweissen unter Wärme vorgenommen werden. Dabei wird das Material der Deckfolie (1) mit dem Material der Membran verschweisst.

Für die Kleberschicht (3) eignen sich die in der Dermatologie verwendbaren Klebematerialien. Geeignete Klebematerialien sind beispielsweise Silikonkleber [z.B. vom Typ des Bio-PSA® oder Silicon Adhesive 355 (Dow Corning)], klebrige Formulierungen von Acrylsäure- oder Methacrylsäureharzen, z.B. Polymere von Acrylsäure oder Methacrylsäure verestert mit Alkoholen wie n-Butanol, n-Pentanol, Isopentanol, 2-Methylbutanol, 1-Methylbutanol, 1-, 2- oder 3-Methylpentanol, 2-Aethylbutanol, Isooctanol, n-Decanol oder n-Dodecanol, oder Copolymerisate dieser Acrylsäure- oder Methacrylsäureester mit Ethylengruppen-haltigen Monomeren, wie Acrylsäure selbst, Methacrylsäure, Acrylamid, Methacrylamid, N-Alkoxymethacrylamid, N-Alkoxymethylmethacrylamid, N-tert-Butylamid, Itaconsäure, Vinylacetat [z.B. vom Typ des Durotak® 280-2516 (National Starch & Chemical B.V.)], N-verzweigtes Alkylmaleinsäureamid, worin die verzweigte Alkylgruppe 10-24 C-Atome hat, Glycoldiacrylate oder Mischungen davon, Polyalkenylene, wie Polyisobutylene mit verschiedenen Molekulargewichten, beispielsweise von etwa 100 bis 1,5 Mio., z.B. Polyisobutylen 300 bzw. 35000 bzw. 1,2 Mio., hydrierte Kohlenwasserstoffharze, natürlicher oder synthetischer Kautschuk, wie Styrolbutadien, Butylether, Neopren, Polyisobutylen, Polybutadien und Polyisopren, Polyvinylacetat, Harnstoff-Formaldehyd-Resinate, Resorcin-Formaldehyd-Resinate, Cellulosederivate wie Ethylcellulose, Methylcellulose, Nitrocellulose, Celluloseacetatbutyrat und Carboxymethylcellulose sowie natürliche Kleber, wie Guar, Acacia, Pektin, Stärke, Dextrin, Albumin, Gelatine, Casein etc. Ebenso geeignet als Klebematerialien sind entsprechende Silikonkleber. Die genannten Klebstoffe können noch mit Verdickern und Stabilisatoren sowie mit Lösungsmitteln versetzt werden; sie können aber lösungsmittelfrei als sog. Schmelzkleber ("hot melt" Kleber) eingesetzt werden, die in Form ihrer Schmelze bei höheren Temperaturen auf die polymeren Materialien aufgebracht werden. Die Lösungsmittel, in denen die Klebematerialien zum Auftragen aus Lösung gelöst werden, sind insbesondere leicht füchtig und hautverträglich. Beispielhaft seien entsprechende Kohlenwasserstoffe, wie Alkane, z.B. Hexan, Heptan, aromatische Kohlenwasserstoffe, z.B. Toluol,

Niederalkanole, wie Methanol, Ethanol oder Isopropanol, Ester, wie Niederalkancarbonsäureniederalkylester, z.B. Ethylacetat, oder Ketone, wie Acetylaceton, oder Gemische davon genannt.

Die Kleberschicht (3) kann teilweise oder vollständig auf der Membran aufgetragen sein. Bei vollständiger Bedeckung der Membran mit Kleberschicht kann diese neben ihrer eigentlichen Funktion als Haftmittel auf der Haut ausserdem als permeable Membran wirken. Die gewünschten Membraneigenschaften, z.B. Steuerung der Diffusionsgeschwindigkeit des Penetrationsverstärkers, lassen sich durch Variation der Stärke und Zusammensetzung der Kleberschicht (3) erzielen. Die Kleberschicht (3) kann ausserdem die gesamte oder vorzugsweise eine anteilige Menge des Wirkstoffs enthalten. Mit der in der Kleberschicht (3) enthaltenen Wirkstoffmenge lässt sich insbesondere eine anfängliche stossweise Zufuhr erzielen, bevor die vom therapeutischen System gesteuerte kontinuierliche Zufuhr auf dem gewünschten therapeutischen Niveau einsetzt.

Die Membran kann ausserdem teilweise und/oder diskontinuierlich mit der Kleberschicht (3) bedeckt sein. Dabei ist eine randförmige Bedeckung möglich, z.B. ringförmige umlaufende Bedeckung. Die Membran kann auch musterförmig bedeckt sein, z.B. rautenförmig. Die Membran kann am äusseren Rand durch ein kontinuierliches Band von Klebematerial, z.B. ringförmig, und auf der Innenfläche mit diskontinuierlichen Bändern, z.B. rautenförmig, bedeckt sein.

Die Schutzfolie (4) wird vor der Applikation entfernt. Sie besteht aus Materialien, die für die Bestandteile der Reservoirschicht (2) undurchlässig sind. Dabei lassen sich dieselben Materialien, die zur Herstellung der Deckfolie (1) dienen können, sowie Metallfolien, z.B. dünne Aluminiumfolie, verwenden. Organische Polymere werden z.B. durch eine abhäsive Oberflächenbehandlung, z.B. Siliconbehandlung, von der Kleberschicht abziehbar gemacht.

Die im erfindungsgemässen transdermalen therapeutischen System, insbesondere im Reservoir (2), enthaltene Wirkstoffzubereitung enthält als Hilfsstoff ein die perkutane Absorption verstärkendes Mittel (Penetrationsverstärker "Flux-enhancer"), welches die Durchflussgeschwindigkeit (flux) der Wirkstoffe der Formel I durch die Haut erhöht, so dass eine grössere Menge an Wirkstoffen bezogen auf die Applikationsfläche und Zeiteinheit von der Haut absorbiert wird. Der Penetrationsverstärker kann ausserdem den Durchgang des Wirkstoffs durch die permeable Membran in Membransystemen beschleunigen. Insbesondere wird bei Verwendung eines geeigneten Penetrationsverstärkers die zur Einhaltung des therapeutischen Niveaus notwendige Dosismenge an Wirkstoffen pro Zeiteinheit durch die Haut appliziert. Penetrationsverstärker sind Stoffe, die, ohne die Haut permanent zu schädigen, ihre Durchlässigkeit für den Wirkstoff erhöhen und mit weiteren pharmazeutisch annehmbaren Hilfsstoffen vermischt sein können.

Geeignete Penetrationsverstärker sind vorzugsweise monovalente, gesättigte oder ungesättigte aliphatische, cycloaliphatische oder aromatische Alkohole mit 4 bis 12 C-Atomen, z.B. n-Hexanol oder Cyclohexanol, aliphatische, cycloaliphatische oder aromatische Kohlenwasserstoffe mit 5 bis 12 C-Atomen, z.B. Hexan, Cyclohexan, Isopropylbenzol u.ä., cycloaliphatische oder aromatische Aldehyde und Ketone mit 4 bis 10 C-Atomen wie Cyclohexanon, Acetamid, N,N-Diniederalkylacetamid wie N,N-Dimethyl- oder N,N-Diethylacetamid, $C_{10}$-$C_{20}$-Alkanoylamide, z.B. N,N-Dimethyllauroylamid, 1-n-$C_{10}$-$C_{20}$-Alkylazacycloheptan-2-on, z.B. 1-n-Dodecylazacycloheptan-2-on (Azone/ , Nelson), Pyrrolidone, wie N-Methyl-pyrrolidon, Polyalkylenglykollaureate, z.B. Polyethylenglykolmonolaureat oder N-2-Hydroxyethylacetamid, ferner bekannte Transportmittel und/oder Penetrationsverbesserer wie aliphatische, cycloaliphatische und aromatische Ester, N,N-Diniederalkylsulfoxid, ungesättigte Oele, halogenierte oder nitrierte aliphatische, cylcoaliphatische Kohlenwasserstoffe, Salicylate, Polyalkylenglycolsilicate sowie Mischungen davon.

Als Penetrationsverstärker sind insbesondere $C_2$-$C_4$-Alkanole, z.B. Isopropanol oder Isobutanol sowie vor allem Ethanol, bevorzugt.

Die zur Erzielung eines therapeutischen Effekts im therapeutischen System enthaltene Wirkstoffmenge ist von vielen Faktoren abhängig: u.a. von der erforderlichen Mindestdosismenge, der Permeabilität des die Durchtrittsgeschwindigkeit bestimmenden Membranmaterials und der Klebschicht sowie der Zeitdauer, in der das Pflaster auf der Haut oder den Schleimhäuten befestigt ist. Da sich die Wirkstoffabgabe über einen längeren Zeitraum als einen Tag erstrecken soll, besteht eigentlich keine Obergrenze hinsichtlich der maximalen im Pflaster enthaltenen Wirkstoffmengen. Die Mindestwirkstoffmenge wird vom Erfordernis festgelegt, dass ausreichende, z.B. therapeutisch wirksame Wirkstoffmengen im Pflaster enthalten sein müssen, um die erwünschte Abgaberate über den vorgesehenen Zeitraum aufrechtzuerhalten.

Den Wirkstoffen können Hilfsstoffe zugesetzt sein. Geeignet sind Hilfsstoffe wie Wasser, isotonische wässrige Kochsalzlösung, Dextrose in Wasser oder Kochsalzlösung, flüssige Glyceryltriester mit niedermolekularen Fettsäuren, Niederalkanole, natürliche Oele wie Maisöl, Erdnussöl, Sesamöl, Castoröl sowie deren Kondensationsprodukte mit Ethlyenoxid und ähnliche, Kohlenwasserstoffe wie Mineralöl pharmazeutischer Qualität, Silicone, Emulgatoren wie Mono-oder Diglyceride von Fettsäuren, Phosphatidsäurederivate wie Lecithin oder Kephalin, Polyalkylenglycole wie Polyäthylenglycol, wässrige Phasen versetzt mit einem

Quellmittel wie Natriumcarboxymethylcellulose, Natriumalginat, Polyvinylpolypyrrolidon etc., denen noch Dispersionsmittel oder Emulgatoren wie Lecithin zugesetzt sein können, Polyoxyethylen und ähnliche. Die Hilfsstoffe können ferner Zusätze wie Konservierungsmittel, Stabilisatoren, Benetzungsmittel, Emulgatoren etc. enthalten.

Bei Verwendung von $C_2$-$C_4$-Alkanolen wie Ethanol als Penetrationsverstärker werden als Hilfsstoffe vorzugsweise Geliermittel, wie Gelatine, oder Quellmittel, wie Celluloseäther, z.B. Hydroxypropylcellulose der Wirkstoffzubereitung zugesetzt.

Die erfindungsgemässen transdermalen therapeutischen Systeme werden auf an sich bekannte Weise hergestellt, z.B. indem man die Kleberschicht (3) auf einer Grundlage (abziehbare Schutzfolie (4)), z.B. Folie oder Film, aufträgt. Auf die Grundlage können auch die Bestandteile des Wirkstoffreservoirs, z.B. Membran und Wirkstoffzubereitung, aufgetragen und darauf die undurchlässige Deckfolie gelegt werden. Anschliessend wird das Pflaster aus der Vorlage ausgestanzt. Gegebenenfalls wird das Reservoir mittels zusätzlichem Kleber mit der Deckfolie verbunden. Ebenso kann das Reservoir mit der Membran oder mit der Kleberschicht heiss verschweisst werden. Bei flüssigkeitsgefüllten Systemen wird die Membran auf die Kleberschicht aufgetragen und auf die Membran die Wirkstoffzubereitung gebracht.

Die Herstellungsverfahren sind in der U.S. Patentschrift Nr. 3,797,494 beschrieben, vorzugsweise in der DE-A-26 04 718 und DE-A-32 05 258 bzw. in den U.S. Patentschriften 4,031,894 und 4,262,003 oder in der Publikation von H. Asche in Schweiz. Rundschau Med. (Praxis) 74, Nr. 11,257-260 (1985), wobei die erfindungsgemässe Anwendung nicht auf die in diesen Publikationen beschriebenen transdermalen therapeutischen Systeme beschränkt ist. Das bevorzugte transdermale therapeutische System gemäss DE-A 32 05 258 ist ein therapeutisches System in Form einer pflasterartigen Auflage, das den Wirkstoff transdermal unter Vermeidung von Nebenwirkungen freisetzt und durch die Haut abgibt, so dass der Wirkstoffgehalt im Plasma annähernd konstant aufrechterhalten bleibt.

Als tägliche Wirkstoffdosierung wird für einen etwa 70 kg schweren Patienten die Verabreichung von etwa 50 µg bis etwa 150 µg, abhängig von der jeweiligen Wirkungsstärke der eingesetzten Wirksubstanz, veranschlagt.

Die nachfolgenden Beispiele illustrieren die vorstehend beschriebene Erfindung, ohne ihren Umfang in irgend einer Weise einzuschränken.

| Beispiel 1: Gel, enthaltend als Wirkstoff z.B. 1-Hydroxy-2-(imidazol-1-yl)-ethan-1,1-diphosphonsäure, | |
|---|---|
| mit folgender Zusammensetzung: | |
| Wirkstoff | 1,0 g |
| Carbopol 934 P | 1,0 g |
| Glycerin | 3,0 g |
| Isopropanol | 25,0 g |
| Softigen 767 | 0,2 g |
| Wasser entmin. q.s. ad | 100,0 g |

| Beispiel 2: Lösung, enthaltend als Wirkstoff z.B. 1-Hydroxy-2-(imidazol-1-yl)-ethan-1,1-diphosphonsäure, | |
|---|---|
| mit folgender Zusammensetzung: | |
| Wirkstoff | 1,0 g |
| Isopropanol | 60,0 g |
| Propylenglykol | 10,0 g |
| Wasser entmin. q.s. ad | 100,0 g |

| Beispiel 3: Mikroemulsion, enthaltend als Wirkstoff z.B. 1-Hydroxy-2-(imidazol-1-yl)-ethan--1,1-diphosphonsäure, | |
|---|---|
| mit folgender Zusammensetzung: | |
| Wirkstoff | 1,0 g |
| Labrasol* | 32,9 g |
| Plurolisostearat** | 13,2 g |
| Isostearylisostearat | 41,9 g |
| Wasser entmin. q.s. ad | 100,0 g |

* Glyceryl caprylat.caprat (und) PEG-8
**Polyglycerol-6-isostearat

| Beispiel 4: Crème (W/O), enthaltend als Wirkstoff z.B. 1-Hydroxy-2-(imidazol-1-yl)-ethan--1,1-diphosphonsäure, | |
|---|---|
| mit folgender Zusammensetzung: | |
| Wirkstoff | 1,0 g |
| Cetylalkohol | 6,5 g |
| Cetylpalmitat | 5,0 g |
| Stearylalkohol | 6,5 g |
| Vaselin | 5,0 g |
| Glycerin | 12,5 g |
| Natriumlaurylsulfat | 1,0 g |
| Methylparaben | 0,18 g |
| Propylparaben | 0,05 g |
| Wasser entmin. q.s. ad | 100,0 g |

| Beispiel 5: Salbe (O/W-Emulsion), enthaltend als Wirkstoff z.B. 1-Hydroxy-2-(imidazol-1-yl)-ethan--1,1-diphosphonsäure, | |
|---|---|
| mit folgender Zusammensetzung: | |
| Wirkstoff | 1,0 g |
| Cetylalkohol | 3,0 g |
| Glycerin | 6,0 g |
| Methylparaben | 0,18 g |
| Propylparaben | 0,05 g |
| Arlacel 60 | 0,6 g |
| Tween 60 | 4,4 g |
| Stearinsäure | 9,0 g |
| Isopropylpalmitat | 2,0 g |
| Paraffinöl dickflüssig | 10,0 g |
| Wasser entmin. q.s. ad | 100,0 g |

| Beispiel 6: Wasserfreie Salbe, enthaltend als Wirkstoff z.B. 1-Hydroxy-2-(imidazol-1-yl)ethan-1,1-diphosphonsäure, | |
|---|---|
| mit folgender Zusammensetzung: | |
| Wirkstoff | 1,0 g |
| Vaselin | 35,0 g |
| Paraffinöl dickflüssig | 35,0 g |
| Lanette N | 30,0 g |

| Beispiel 7: Lotion, enthaltend als Wirkstoff z.B. 1-Hydroxy-2-(imidazol-1-yl)-ethan--1,1-diphosphonsäure, | |
|---|---|
| mit folgender Zusammensetzung: | |
| Wirkstoff | 1,0 g |
| Cetylalkohol | 1,9 g |
| Glycerin | 4,3 g |
| Natriumlaurylsulfat | 1,0 g |
| Methylparaben | 0,18 g |
| Propylparaben | 0,05 g |
| Paraffinöl dickflüssig | 2,5 g |
| Wasser entmin. q.s. ad | 100,0 g |

| Beispiel 8: Schaum, enthaltend als Wirkstoff z.B. 1-Hydroxy-2-(imidazol-1-yl)-ethan--1,1-diphosphonsäure, | |
|---|---|
| mit folgender Zusammensetzung: | |
| Wirkstoff | 1,0 g |
| Cetylalkohol | 1,7 g |
| Glycerin | 5,0 g |
| Methylparaben | 0,18 g |
| Propylparaben | 0,05 g |
| Isopropylpalmitat | 2,0 g |
| Arlacel 83 | 1,5 g |
| Cetomacrogol 1000 | 2,4 g |
| Paraffinöl dickflüssig | 1,0 g |
| Wasser entmin. q.s. ad | 100,0 g |

| Beispiel 9: Monolithisches Klebermatrix-Transdermalsystem, enthaltend als Wirkstoff z.B. 1-Hydroxy-2-(imidazol-1-yl)-ethan-1,1-diphosphonsäure | |
|---|---|
| Zusammensetzung: | |
| Polyisobutylen (PIB) 300 (Oppanol B1, BASF) | 5,0 g |
| PIB 35000 (Oppanol B10, BASF) | 3,0 g |
| PIB 1200000 (Oppanol B100, BASF) | 9,0 g |
| Hydrogenated hydrocarbon resin (Escorez 5320, Exxon) | 43,0 g |
| 1-Dodecylazacycloheptan-2-on (Azone, Nelson Res., Irvine/C | 20,0 g |
| Wirkstoff | 20,0 g |
| Total | 100,0 g |

Herstellung:

Die obigen Komponenten werden zusammen in 150 g Siedegrenzbenzin 100-125 durch Umwälzen auf dem Rollentisch gelöst. Die Lösung wird mit einem Streichgerät unter Verwendung eines 300 µm Rakels auf eine Polyesterfolie (Hostaphan, Kalle) aufgetragen, was eine Beschichtung von ca. 75 g/m$^2$ ergibt. Nach der Trocknung (15 Minuten bei 60 °C) wird eine silikonisierte Polyesterfolie (Dicke 75 µm, Laufenberg) als Abziehfolie aufkaschiert. Mittels eines Stanzwerkzeugs werden Systeme in Grössen von 5 bis 30 cm$^2$ in gewünschter Form ausgestanzt. Die fertigen Systeme werden einzeln in Sachets aus aluminisiertem Papier eingesiegelt.

| Beispiel 10: Matrix-Transdermalsystem mit Kleberbeschichtung nur am Rand, enthaltend als Wirkstoff z.B. 1-Hydroxy-2-(imidazol-1-yl)-ethan-1,1-diphosphonsäure | |
|---|---|
| Zusammensetzung: | |
| Wirkstoff | 10,0 g |
| Hydroxypropylcellulose (Klucel HF) | 1,0 g |
| Polyethylenglykol-monolaurat | 5,0 g |
| Wasser entmineralisiert | 84,0 g |

Herstellung:

Die Bestandteile werden in Wasser unter Rühren gelöst. Eine Polyesterfolie (Hostaphan, Kalle) wird unter Verwendung eines Streichgeräts und eines 150 µm Rakels mit Silikonkleber (Silicon Adhesive 355, Dow Corning, 55 % Feststoff in Siedegrenzbenzin 100-120 gelöst) beschichtet und getrocknet (15 Minuten 60 °C).

Von einem Vliesmaterial aus Viskose, Polyamid und Polypropylen (Vilmed Typ M 1539, Freudenberg) mit einer Dicke von ca. 2,7 mm werden Scheibchen von 2,5 cm Durchmesser (entsprechend einer Fläche von 5 cm$^2$) ausgestanzt. Nachdem diese auf die beschichtete Seite der Polyesterfolie geklebt wurden, wird die obige Lösung in einer Menge von jeweils 450 mg pro System mit einem Mikrodosiersystem auf die Vliesscheibe zudosiert. Die Abdeckung erfolgt mit einer silikonisierten Polyesterfolie. Die einzelnen Systeme werden mit einem Werkzeug mit Durchmesser 5 cm ausgestanzt, wobei darauf zu achten ist, dass die mit der Wirkstofflösung beladene Vliesscheibe in die Mitte des Systems zu liegen kommt.

Die fertigen Systeme werden wie im Beispiel 9 beschrieben einzeln verpackt.

| Beispiel 11: Membran-Transdermalsystem, mit Siegelnaht am Rand verschlossen, enthaltend als Wirkstoff z.B. 1-Hydroxy-2-(imidazol-1-yl)-ethan-1,1-diphosphonsäure | |
|---|---|
| Zusammensetzung: | |
| Wirkstoff | 10,0 g |
| Hydroxypropylcellulose (Klucel HF) | 1,0 g |
| Polyethylenglykol-monolaurat | 5,0 g |
| Wasser entmineralisiert | 84,0 g |

Herstellung:

Die Kleberschicht (Silikonkleber, siehe Beispiel 10) wird mittels eines Rasterdruckverfahrens auf eine fluorierte Polyesterfolie aufgetragen und getrocknet. Aufkaschieren eines heißsiegelbaren Vliesmaterials bestehend aus Cellulose und Polyester (Sontara Vlies 8412, DuPont), so dass ein Laminat bestehend aus Abziehfolie, Kleberschicht und Vlies resultiert.
Die Lösung wird mittels eines Mikrodosiersystems auf das obige Laminat (Vlies oben) in einer Menge von 300 mg für ein System von 5 $cm^2$ aufgetragen, eine Abdeckfolie bestehend aus eine heißsiegelbaren Polyester/Ethylvinylacetat-Verbundfolie (Scotchpack 1220, 3M) darübergezogen (Ethylvinylacetatseite gegen Lösung, bzw. Vlies), und das System mittels eines geeigneten Heißsiegelwerkzeugs (Innendurchmesser 2,5 cm, Aussendurchmesser 3,5 cm) versiegelt (Siegelbedingungen: 0,5 sec, 170 °C) und anschliessend ausgestanzt und wie in Beispiel 9 einzeln verpackt.

| Beispiel 12: System analog Beispiel 11 | |
|---|---|
| Zusammensetzung: | |
| Wirkstoff | 10,0 g |
| Hydroxypropylcellulose (Klucel HF) | 3,0 g |
| 1-Dodecylazacycloheptan-2-on Azone, Nelson Res., Irvine CA) | 5,0 g |
| Ethanol 50 % | 84,0 g |

Herstellung:

Die Bestandteile werden durch Rühren aufgelöst. Herstellung und Verpackung der Systeme analog Beispiel 11.

Beispiel 13: Zweischichtiges Matrix-Transdermalsystem, enthaltend als Wirkstoff z.B. 1-Hydroxy-2-(imidazol-1-yl)-ethan-1,1-diphosphonsäure

Schicht 1: Identisch mit der Matrix von Beispiel 9, wobei diese mit einem 150-250 μm Rakel (Auftragsmenge 30-60 $g/m^2$) auf eine silikonisierte Polyesterfolie (Spezifikationen siehe Beispiel 9) aufgetragen und mit einer silikonisierten Papierfolie (Zwischenabdeckung) abgedeckt wird.

Schicht 2: In der in Beispiel 9 spezifizierten Klebermasse ohne Wirkstoff (gelöst in Benzin) bzw. in einem andern geeigneten Adhäsiv (z.B. Copolymer auf der Basis von Acrylatestern und Vinylacetat, Durotak 280-2516, National Starch & Chemical BV, Zutphen/NL) wird eine geeignete Menge des Penetrationsverstärkers (z.B. 5-25 % 1-Dodecylazacycloheptan-2-on, Azone) gelöst. Die Klebermasse wird mittels Rakel auf einer Polyesterfolie (mit oder ohne Aluminiumbeschichtung) in einer Schichtdicke von 150-300 μm unter

gleichzeitigem Abziehen und Verwerfen der Zwischenabdeckung auflaminiert. Die weitere Verarbeitung erfolgt wie in Beispiel 9.

Beispiel 14: Zweischichtiges Matrix-Transdermalsystem, enthaltend als Wirkstoff z.B. 1-Hydroxy-2-(imidazol-1-yl)-ethan-1,1-diphosphonsäure

Schicht 1: Wie Beispiel 13.

Schicht 2: Eine geeignete Menge des Penetrationsverstärkers wird in einen Schmelzkleber ( z.B. Acrylatkleber Durotak 089-1526) eingearbeitet. Mit diesem Gemisch wird eine aluminiumbedampfte oder -kaschierte Polyesterfolie mittels einer Schmelzauftragevorrichtung bei ca. 140-160 °C beschichtet. Die weitere Verarbeitung der Schichten 1 und 2 zum Laminat sowie die Herstellung und Verpackung erfolgt wie in Beispiel 13.

Beispiel 15: Membran-Transdermalsystem (Reservoir-Typ), enthaltend als Wirkstoff z.B. 1-Hydroxy-2-(imidazol-1-yl)-ethan-1,1-diphosphonsäure

Zusammensetzung analog Beispiel 11.

Auf die Membranseite eines Laminats bestehend aus Abziehfolie (Polyester, einseitig silikonisiert, Dicke ca. 75 µm); Kleberschicht (z.B. geeignete Mischung aus Polyisobutylen 1200000, Polyisobutylen 35000 und Polyisobutylen 1200; Auftragsmenge 40-60 g/m$^2$); Steuermembran (Ethylvinylacetat mit 6-25 % Vinylacetat, vorzugsweise 12-18 %, Dicke ca. 50 µm) wird in einer geeigneten Anlage je nach vorgesehener Systemfläche ca. 100-600 mg der Reservoirzubereitung gemäss Beispiel 12 aufdosiert (auf Membranseite des Laminats), mit einer Schutzfolie bestehend aus Polyester und Ethyl-vinylacetat bedeckt, bei ca. 130 °C formgesiegelt und anschliessend die einzelnen Systeme ausgestanzt und verpackt.

Alternativ dazu kann der Wirkstoff, bzw. ein Teil davon, in der Kleberschicht gelöst, bzw. suspendiert werden. Dabei würde der in der Kleberschicht enthaltene Wirkstoff als Initial-und derjenige im Reservoir als Erhaltungsdosis wirken.

In analoger Weise wie in einem der vorstehenden Beispiele kann man andere Wirkstoffe der Formel I in die Grundlagen einarbeiten, beispielsweise ausgewählt aus 4-Amino-1-hydroxy-butan-1,1-diphosphonsäure, 6-Amino-1-hydroxy-hexan-1,1-diphosphonsäure, 3-(N-Methyl-N-n-pentyl-amino)-1-hydroxy-propan-1,1-diphosphon säure, 3-[N-(3-Phenylpropyl)-N-methyl-amino]-1-hydroxy-propan-1,1-diphos phonsäure, 3-(N-Methyl-N-5-phenylpentyl-amino)-1-hydroxypropan-1,1-diphosphonsäure, 3-[N-Methyl-N-3-(2-pyridyl)-propyl-amino]-1-hydroxy-propan-1,1-diphosphonsäure, 1-Hydroxy-3-[N-methyl-N-(3-phenoxypropyl)-amino]-propan-1,1-diphosphonsäure, 1-Hydroxy-3-[N-methyl-N-(2-phenoxyethyl)-amino]-propan-1,1-diphosphonsäure, 4-(4-Phenyl-piperidin-1-yl)-1-hydroxy-butan-1,1-diphosphonsäure, 1-Hydroxy-3-(1-piperidino)-propan-1,1-diphosphonsäure, 1-Hydroxy-3-[3-(4-chlorphenyl)-pyrrolidin-1-yl]-propan-1,1-diphosphonsäure, 1-Hydroxy-2-(imidazol-1-yl)-ethan-1,1-diphosphonsäure, 2-(1-Methyl-imidazol-2-yl)-ethan-1,1-diphosphonsäure, 1-Hydroxy-2-(4-methyl-imidazol-5-yl)-ethan-1,1-diphosphonsäure, 1-Hydroxy-2-(imidazol-5-yl)-ethan-1,1-diphosphonsäure, 1-Hydroxy-2-(3-pyridyl)-ethan-1,1-diphosphonsäure, 2-(2-Pyridyl)-ethan-1,1-diphosphonsäure, 1-[(5-n-Butyl-2-thiazolyl)-amino]-methan-1,1-diphosphonsäure,1-[(5-Methyl-2-thiazolyl)-amino]-methan-1,1-diphosphonsäure,1-[(2-Thiazolyl)-amino]-methan-1,1-diphosphonsäure, 1-Hydroxy-ethan-1,1-diphosphonsäure, 1 -(4-Chlor-phenylthio)-methan-1,1-diphosphonsäure, 1,1-Dichlormethan-1,1-diphosphonsäure oder 3-(1,5-Dimethyl-3-aza-bicyclo[3.1.1]hept-3-yl)-1-hydroxy-propan-1,1-diphosphonsäure, ebenso 1-[(5-Ethyl-2-thiazolyl)-amino]-methan-1,1-diphosphonsäure, 3-[N-(2-Phenylethyl)-N-methyl-amino]-1-hydroxy-propan-1,1-dip hosphonsäure, 3-[N-(2-Phenylthioethyl)-N-methyl-amino]-1-hydroxy-propan-1,1-diphosphonsäure, 1-Hydroxy-3-(pyrrolidin-1-yl)-propan-1,1-diphosphonsäure oder 2-(N-Cycloheptylamino)-ethan-1,1-diphosphonsäure oder ein pharmazeutisch verwendbares Salz davon.

## Ansprüche

1. Topisch applizierbare pharmazeutische Präparate enthaltend pharmazeutisch verwendbare Methandiphosphonsäure-Derivate der Formel

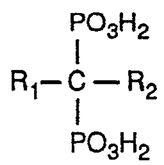

(I)

und ihre Salze, worin $R_1$ und $R_2$ jeweils Halogen bedeuten, oder worin $R_1$ Wasserstoff ist und $R_2$ Ar-S- oder $Het_1$-NH- bedeutet und Ar gegebenenfalls substituiertes Phenyl bedeutet und $Het_1$ gegebenenfalls substituiertes, monocyclisches, 5- oder 6-gliedriges Monoaza-, Diaza- oder Thiazaaryl bedeutet, welches über ein Ringkohlenstoffatom gebunden ist, oder worin $R_1$ Wasserstoff oder Hydroxy bedeutet und $R_2$ für -A-$R_3$ steht, worin A Alkylen bedeutet, und einerseits $R_3$ für $Het_2$ steht, das über ein Ringkohlenstoff-oder Ringstickstoffatom gebundenes $Het_1$ bedeutet, oder andererseits $R_3$ Wasserstoff oder gegebenenfalls durch Alkyl, Cycloalkyl, Ar-Alkyl, Ar-O-Alkyl, Ar-S-Alkyl oder $Het_1$-Alkyl unabhängig voneinander mono- oder disubstituiertes oder durch gegebenenfalls Ar enthaltendes Alkylen disubstituiertes Amino bedeutet, wobei zwei Alkylenkohlenstoffatome zusätzlich über Alkylen miteinander verknüpft sein können und Ar bzw. $Het_1$ jeweils die vorstehend angegebenen Bedeutungen hat, mit der Massgabe, dass, wenn $R_2$ für -A-$R_3$ steht, A Ethylen bedeutet und $R_3$ gegebenenfalls durch $C_1$-$C_3$-Alkyl substituiertes Amino bedeutet, $R_1$ von Hydroxy verschieden ist.

2. Präparate gemäss Anspruch 1, enthaltend eine Verbindung der Formel I oder ein Salz davon enthalten, worin $R_1$ und $R_2$ jeweils Halogen bedeuten, oder worin $R_1$ Wasserstoff ist und $R_2$ Ar-S- oder $Het_1$-NH-bedeutet und Ar gegebenenfalls substituiertes Phenyl bedeutet und $Het_1$ gegebenenfalls substituiertes, monocyclisches, 5- oder 6-gliedriges Monoaza-, Diaza- oder Thiazaaryl bedeutet, welches über ein Ringkohlenstoffatom gebunden ist, oder worin $R_1$ Wasserstoff oder Hydroxy bedeutet und $R_2$ für -A-$R_3$ steht, worin A Alkylen bedeutet, und einerseits $R_3$ für $Het_2$ steht, das über ein Ringkohlenstoff-oder Ringstickstoffatom gebundenes $Het_1$ bedeutet, oder andererseits $R_3$ Wasserstoff oder gegebenenfalls durch Alkyl, Ar-Alkyl, Ar-O-Alkyl oder $Het_1$-Alkyl unabhängig voneinander mono- oder disubstituiertes oder durch gegebenenfalls Ar enthaltendes Alkylen disubstituiertes Amino bedeutet, wobei zwei Alkylenkohlenstoffatome zusätzlich über Alkylen miteinander verknüpft sein können und Ar bzw. $Het_1$ jeweils die vorstehend angegebenen Bedeutungen hat.

3. Präparate gemäss Anspruch 2, enthaltend eine Verbindung der Formel I oder ein Salz davon enthalten, worin $R_1$ und $R_2$ jeweils Halogen bedeuten, oder worin $R_1$ Wasserstoff ist und $R_2$ Ar-S- oder $Het_1$-NH-bedeutet und Ar gegebenenfalls durch Niederalkyl, Niederalkoxy, Trifluormethyl und/oder insbesondere Halogen substituiertes Phenyl bedeutet, und $Het_1$ unsubstituiertes oder durch Niederalkyl substituiertes Thiazolyl bedeutet, oder worin $R_1$ Wasserstoff oder Hydroxy bedeutet und $R_2$ für -A-$R_3$ steht, worin A Niederalkylen bedeutet und einerseits $R_3$ gegebenenfalls durch Niederalkyl substituiertes Imidazolyl, welches über ein Ringkohlenstoff- oder ein Ringstickstoffatom gebunden ist, oder Pyridyl bedeutet, oder andererseits $R_3$ Wasserstoff oder gegebenenfalls durch Niederalkyl, Ar-Niederalkyl, Ar-O-Niederalkyl oder Pyridylniederalkyl unabhängig voneinander mono- oder disubstituiertes oder durch gegebenenfalls Ar enthaltendes Niederalkylen oder durch Niederalkylen, worin zwei nicht benachbarte Niederalkylenkohlenstoffatome zusätzlich über Niederalkylen miteinander verknüpft sind, disubstituiertes Amino bedeutet, und Ar jeweils die vorstehend angegebenen Bedeutungen hat.

4. Präparate gemäss Anspruch 3 enthaltend eine Verbindung der Formel I oder ein Salz davon, worin $R_1$ Wasserstoff oder Hydroxy bedeutet und $R_2$ für -A-$R_3$ steht, worin A Niederalkylen bedeutet und $R_3$ durch Niederalkyl, Cycloalkyl, Ar-Niederalkyl, Ar-O-Niederalkyl, Ar-S-Niederalkyl oder Pyridylniederalkyl unabhängig voneinander mono- oder disubstituiertes Amino bedeutet.

5. Präparate gemäss Anspruch 2 enthaltend eine Verbindung der Formel I oder ein Salz, insbesondere Alkalimetall-, z.B. Natriumsalz, davon enthalten, worin $R_1$ und $R_2$ jeweils Halogen, insbesondere Chlor, bedeuten, oder worin $R_1$ Wasserstoff ist und $R_2$ gegebenenfalls durch Halogen, wie Chlor, substituiertes Phenylthio, wie 4-Chlorphenylthio, oder gegebenenfalls durch Niederalkyl, insbesondere $C_1$-$C_4$-Alkyl, wie Methyl, substituiertes Thiazolylamino, wie Thiazol-2-yl-, 5-n-Butylthiazol-2-yl- oder 5-Methylthiazol-2-yl-amino, bedeutet, oder worin $R_1$ Wasserstoff oder Hydroxy bedeutet und $R_2$ für -A-$R_3$ steht, worin A $C_1$-$C_7$Alkylen, wie Methylen, Ethylen, Propylen oder Pentylen, bedeutet und $R_3$ ein über ein Ringkohlenstoff- oder Ringstickstoffatom gebundenes, gegebenenfalls durch Niederalkyl, insbesondere $C_1$-$C_4$-Alkyl, wie Methyl, substituiertes Imidazolyl, wie Imidazol-1-yl, Imidazol-5-yl, 1-Methyl-imidazol-2-yl oder 4-Methyl-imidazol-5-yl, oder Pyridyl, wie 2- oder 3-Pyridyl, bedeutet, oder worin $R_1$ Hydroxy ist und $R_2$ für -A-$R_3$ steht, worin A die oben angegebene Bedeutung hat und $R_3$ Amino, Di-$C_1$-$C_5$-alkylamino, wie Dimethyl-, N-Methyl-N-n-propylamino oder N-Methyl-N-n-pentylamino, N-$C_1$-$C_4$-Alkyl-N-phenyl-$C_1$-$C_5$-alkylamino, wie N-

Methyl-N-(3-phenylpropyl)-amino oder N-Methyl-N-(5-phenylpentyl)-amino, N-$C_1$-$C_4$-Alkyl-N-pyridyl-$C_1$-$C_4$-alkylamino, wie N-Methyl-N-[3-(2-pyridyl)-propyl]-amino, $C_4$-$C_6$-Alkylenamino, welches gegebenenfalls durch gegebenenfalls Halogen, wie Chlor, enthaltendes Phenyl, wie 4-Chlorphenyl, substituiert ist, z.B. Piperidin-1-yl, welches gegebenenfalls, insbesondere in Position 4, durch Phenyl substituiert ist, oder Pyrrolidin-1-yl, welches gegebenenfalls, insbesondere in Position 3, durch 4-Chlorphenyl substituiert ist, oder 1,5-Di-$C_1$-$C_4$-alkyl-, wie 1,5-Dimethyl-3-aza-bicyclo[3.1.1]hept-3-yl bedeutet, oder worin $R_1$ Hydroxy ist und $R_2$ für -A-$R_3$ steht, worin A $C_1$-$C_4$-Alkylen, insbesondere Methylen, bedeutet und $R_3$ Wasserstoff ist.

6. Präparate gemäss Anspruch 5 enthaltend eine Verbindung der Formel I oder ein Salz davon, worin $R_1$ Wasserstoff oder Hydroxy bedeutet und $R_2$ für -A-$R_3$ steht, worin A $C_1$-$C_7$-Alkylen, wie Methylen, Ethylen, Propylen oder Pentylen, bedeutet und $R_3$ N-$C_3$-$C_7$-Cycloalkylamino, wie N-Cycloheptylamino, oder N-$C_1$-$C_4$-Alkyl-N-phenylthio-$C_1$-$C_4$-alkyl, wie N-Methyl-N-(2-phenylthioethyl)-amino, bedeutet.

7. Präparate gemäss Anspruch 2 enthaltend eine Verbindung der Formel I oder ein Salz, insbesondere Alkalimetall-, z.B. Natriumsalz, davon enthalten, worin $R_1$ Hydroxy ist und $R_2$ für -A-$R_3$ steht, worin A $C_2$-$C_7$-Alkylen, wie Ethylen, Propylen oder Pentylen, bedeutet und $R_3$ Amino, Di-$C_1$-$C_4$-alkylamino, wie Dimethyl- oder N-Methyl-N-n-propylamino, oder N-$C_1$-$C_4$-Alkyl-N-phenyl-$C_1$-$C_4$-alkylamino, wie N-Methyl-N-(3-phenylpropyl)-amino, bedeutet.

8. Präparate gemäss Anspruch 2 enthaltend eine Verbindung der Formel I oder ein Salz davon, insbesondere Alkalimetall-, z.B. Natriumsalz, davon enthalten, worin $R_1$ Hydroxy ist und $R_2$ für -A-$R_3$ steht, worin A $C_1$-$C_7$-Alkylen, insbesondere $C_1$-$C_4$-Alkylen, wie Methylen, bedeutet und $R_3$ ein über ein Ringkohlenstoff- oder Ringstickstoffatom gebundenes, gegebenenfalls durch $C_1$-$C_4$-Alkyl, wie Methyl, substituiertes Imidazolyl, wie Imidazol-1-yl, Imidazol-5-yl, 1-Methyl-imidazol-2-yl oder 4-Methyl-imidazol-5-yl, bedeutet.

9. Präparate gemäss Anspruch 2 enthaltend eine pharmazeutisch wirksame Methandiphos phonsäure ausgewählt aus folgenden Verbindungen der Formel I: 4-Amino-1-hydroxybutan-1,1-diphosphonsäure, 6-Amino-1-hydroxy-hexan-1,1-diphosphonsäure, 3-(N-Methyl-N-n-pentyl-amino)-1-hydroxy-propan-1,1-diphosphonsäure, 3-[N-(3-Phenylpropyl)-N-methyl-amino]-1-hydroxy-propan-1,1-diphosphonsäure, 3-(N-Methyl-N-5-phenylpentyl-amino)-1-hydroxypropan-1,1-diphosphonsäure,3-[N-Methyl-N-3-(2-pyridyl)-propyl-amino]-1-hydroxy-propan-1,1-diphosphonsäure, 1-Hydroxy-3-[N-methyl-N-(3-phenoxypropyl)-amino]-propan-1,1-diphosphonsäure,1-Hydroxy-3-[N-methyl-N-(2-phenoxyethyl)-amino]-propan-1,1-diphosphonsäure, 4-(4-Phenyl-piperidin-1-yl)-1-hydroxy-butan-1,1-diphosphonsäure,1-Hydroxy-3-(1-piperidino)-propan-1,1-diphosphonsäure, 1-Hydroxy-3-[3-(4-chlorphenyl)-pyrrolidin-1-yl]-propan-1,1-diphosphonsäure, 1-Hydroxy-2-(imidazol-1-yl)-ethan-1,1-diphosphonsäure,2-(1-Methyl-imidazol-2-yl)-ethan-1,1-diphosphonsäure, 1-Hydroxy-2-(4-methyl-imidazol-5-yl)-ethan-1,1-diphosphonsäure, 1-Hydroxy-2-(imidazol-5-yl)-ethan-1,1-diphosphonsäure, 1-Hydroxy-2-(3-pyridyl)-ethan-1,1-diphosphonsäure, 2-(2-Pyridyl)-ethan-1,1-diphosphonsäure, 1-[(5-n-Butyl-2-thiazolyl)-amino]-methan-1,1-diphosphonsäure, 1-[(5-Methyl-2-thiazolyl)-amino]-methan-1,1-diphosphonsäure, 1-[(2-Thiazolyl)-amino]-methan-1,1-diphosphonsäure, 1-Hydroxy-ethan-1,1-diphosphonsäure,1-(4-Chlor-phenylthio)-methan-1,1-diphosphonsäure, 1,1-Dichlormethan-1,1-diphosphonsäure oder 3-(1,5-Dimethyl-3-aza-bicyclo[3.1.1]hept-3-yl)-1-hydroxy-propan-1,1-diphosphonsäure oder ein pharmazeutisch verwendbares Salz davon.

10. Präparate gemäss Anspruch 1 enthaltend eine pharmazeutisch wirksame Methandiphosphonsäure ausgewählt aus folgenden Verbindungen der Formel I: 1-[(5-Ethyl-2-thiazolyl)-amino]-methan-1,1-diphosphonsäure, 3-[N-(2-Phenylethyl)-N-methyl-amino]-1-hydroxy-propan-1,1-diphosphonsäure, 3-[N-(2-Phenylthioethyl)-N-methyl-amino]-1-hydroxy-propan-1,1-diphosphonsäure, 1-Hydroxy-3-(pyrrolidin-1-yl)-propan-1,1-diphosphonsäure, 2-(N-Cycloheptylamino)-ethan-1,1-diphosphonsäure oder ein pharmazeutisch verwendbares Salz davon.

11. Präparate gemäss Anspruch 2 enthaltend 1-Hydroxy-2-(imidazol-1-yl)-ethan-1,1-diphosphonsäure oder ein pharmazeutisch verwendbares Salz davon.

12. Präparate gemäss einem der Ansprüche 1-11 in Form von Cremen, Gelen, Salben, Lotionen, Pasten, Schäumen, Tinkturen, Mikroemulsionen oder Lösungen.

13. Präparate gemäss einem der Ansprüche 1-11 in Form mehrschichtiger therapeutischer Systeme für die transdermale Applikation.

14. Präparate gemäss Anspruch 13, worin die mehrschichtigen therapeutischen Systeme für die transdermale Applikation folgende Bestandteile enthalten:

(1) eine geschlossene und für die Bestandteile der nachfolgenden Schichten der Wirkstoffzubereitung undurchlässige Deckfolie,

(2) ein an die Deckfolie (1) einseitig anschliessendes Reservoir für den Wirkstoff, falls dieser nicht in die Kleberschicht eingearbeitet ist,

(3) eine Kleberschicht und

(4) eine abziehbare Schutzfolie.

15. Präparate gemäss einem der Ansprüche 1-11, 13 und 14 in Form mehrschichtiger therapeutischer Systeme für die transdermale Applikation vom Matrix- oder vom Membran-Typ.
16. Verfahren zur Steigerung der Resorption von pharmazeutisch verwendbaren Methandiphosphonsäure-Derivaten der Formel I, welches dadurch gekennzeichnet ist, dass man eine Verbindung der Formel I oder ein Salz davon in eine pharmazeutische Zusammensetzung zur topischen Applikation einarbeitet.
17. Verwendung von topisch applizierbaren pharmazeutischen Präparaten gemäss einem der Ansprüche 1-15 zur Behandlung von Hypercalcämie und osteolytischen Knochenmetastasen.

Patentansprüche für folgende Vertragsstaaten: ES,GR

1. Verfahren zur Herstellung von topisch applizierbaren pharmazeutischen Präparaten enthaltend pharmazeutisch verwendbare Methandiphosphonsäure-Derivate, dadurch gekennzeichnet, dass
A.) pharmazeutisch verwendbare Methandiphosphonsäure-Derivate der Formel

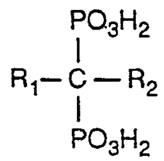

(I)

und ihre Salze, worin $R_1$ und $R_2$ jeweils Halogen bedeuten, oder worin $R_1$ Wasserstoff ist und $R_2$ Ar-S- oder $Het_1$-NH- bedeutet und Ar gegebenenfalls substituiertes Phenyl bedeutet und $Het_1$ gegebenenfalls substituiertes, monocyclisches, 5- oder 6-gliedriges Monoaza-, Diaza- oder Thiazaaryl bedeutet, welches über ein Ringkohlenstoffatom gebunden ist, oder worin $R_1$ Wasserstoff oder Hydroxy bedeutet und $R_2$ für -A-$R_3$ steht, worin A Alkylen bedeutet, und einerseits $R_3$ für $Het_2$ steht, das über ein Ringkohlenstof-oder Ringstickstoffatom gebundenes $Het_1$ bedeutet, oder andererseits $R_3$ Wasserstoff oder gegebenenfalls durch Alkyl, Cycloalkyl, Ar-Alkyl, Ar-O-Alkyl, Ar-S-Alkyl oder $Het_1$-Alkyl unabhängig voneinander mono- oder disubstituiertes oder durch gegebenenfalls Ar enthaltendes Alkylen disubstituiertes Amino bedeutet, wobei zwei Alkylenkohlenstoffatome zusätzlich über Alkylen miteinander verknüpft sein können und Ar bzw. $Het_1$ jeweils die vorstehend angegebenen Bedeutungen hat, mit der Massgabe, dass, wenn $R_2$ für -A-$R_3$ steht, A Ethylen bedeutet und $R_3$ gegebenenfalls durch $C_1$-$C_3$-Alkyl substituiertes Amino bedeutet, $R_1$ von Hydroxy verschieden ist, und
B.) pharmazeutisch verwendbare Hilfs- und/oder Zusatzstoffe, die für eine topische Verwendung geeignet sind,
zu entsprechenden topisch applizierbaren pharmazeutischen Präparaten verarbeitet.
2. Verfahren gemäss Anspruch 1, dadurch gekennzeichnet, dass ein pharmazeutisch verwendbares Methandiphosphonsäure-Derivat der Formel I oder ein Salz davon, worin $R_1$ und $R_2$ jeweils Halogen bedeuten, oder worin $R_1$ Wasserstoff ist und $R_2$ Ar-S- oder $Het_1$-NH- bedeutet und Ar gegebenenfalls substituiertes Phenyl bedeutet und $Het_1$ gegebenenfalls substituiertes, monocyclisches, 5- oder 6-gliedriges Monoaza-, Diaza- oder Thiazaaryl bedeutet, welches über ein Ringkohlenstoffatom gebunden ist, oder worin $R_1$ Wasserstoff oder Hydroxy bedeutet und $R_2$ für -A-$R_3$ steht, worin A Alkylen bedeutet, und einerseits $R_3$ für $Het_2$ steht, das über ein Ringkohlenstoff- oder Ringstickstoffatom gebundenes $Het_1$ bedeutet, oder andererseits $R_3$ Wasserstoff oder gegebenenfalls durch Alkyl, Ar-Alkyl, Ar-O-Alkyl oder $Het_1$-Alkyl unabhängig voneinander mono- oder disubstituiertes oder durch gegebenenfalls Ar enthaltendes Alkylen disubstituiertes Amino bedeutet, wobei zwei Alkylenkohlenstoffatome zusätzlich über Alkylen miteinander verknüpft sein können und Ar bzw. $Het_1$ jeweils die vorstehend angegebenen Bedeutungen hat, eingearbeitet wird.
3. Verfahren gemäss Anspruch 1, dadurch gekennzeichnet, dass ein pharmazeutisch verwendbares Methandiphosphonsäure-Derivat der Formel I oder ein Salz davon, worin $R_1$ und $R_2$ jeweils Halogen bedeuten, oder worin $R_1$ Wasserstoff ist und $R_2$ Ar-S- oder $Het_1$-NH-bedeutet und Ar gegebenenfalls durch Niederalkyl, Niederalkoxy, Trifluormethyl und/oder insbesondere Halogen substituiertes Phenyl bedeutet, und $Het_1$ unsubstituiertes oder durch Niederalkyl substituiertes Thiazolyl bedeutet, oder worin $R_1$ Wasserstoff oder Hydroxy bedeutet und $R_2$ für -A-$R_3$ steht, worin A Niederalkylen bedeutet und einerseits $R_3$ gegebenenfalls durch Niederalkyl substituiertes Imidazolyl, welches über ein Ringkohlenstoff- oder ein Ringstickstoffatom gebunden ist, oder Pyridyl bedeutet, oder andererseits $R_3$ Wasserstoff oder gegebenenfalls durch Niederalkyl, Ar-Niederalkyl, Ar-O-Niederalkyl oder Pyridylniederalkyl unabhängig voneinander mono- oder disubstituiertes oder durch gegebenenfalls Ar enthaltendes Niederalkylen oder durch Niederalkylen, worin zwei nicht benachbarte Niederalkylenkohlenstoffatome zusätzlich über Niederalkylen miteinander verknüpft sind, disubstituiertes Amino bedeutet, und Ar jeweils die vorstehend angegebenen Bedeutungen hat, eingearbeitet wird.

4. Verfahren gemäss Anspruch 1, dadurch gekennzeichnet, dass ein pharmazeutisch verwendbares Methandiphosphonsäure-Derivat der Formel I oder ein Salz davon, worin $R_1$ Wasserstoff oder Hydroxy bedeutet und $R_2$ für $-A-R_3$ steht, worin A Niederalkylen bedeutet und $R_3$ durch Niederalkyl, Cycloalkyl, Ar-Niederalkyl, Ar-O-Niederalkyl, Ar-S-Niederalkyl oder Pyridylniederalkyl unabhängig voneinander mono- oder disubstituiertes Amino bedeutet, eingearbeitet wird.

5. Verfahren gemäss Anspruch 1, dadurch gekennzeichnet, dass ein pharmazeutisch verwendbares Methandiphosphonsäure-Derivat der Formel I oder ein Salz davon, worin $R_1$ und $R_2$ jeweils Halogen, insbesondere Chlor, bedeuten, oder worin $R_1$ Wasserstoff ist und $R_2$ gegebenenfalls durch Halogen, wie Chlor, substituiertes Phenylthio, wie 4-Chlorphenylthio, oder gegebenenfalls durch Niederalkyl, insbesondere $C_1$-$C_4$-Alkyl, wie Methyl substituiertes Thiazolylamino, wie Thiazol-2-yl-, 5-n-Butylthiazol-2-yl- oder 5-Methylthiazol-2-ylamino, bedeutet, oder worin $R_1$ Wasserstoff oder Hydroxy bedeutet und $R_2$ für $-A-R_3$ steht, worin A $C_1$-$C_7$Alkylen, wie Methylen, Ethylen, Propylen oder Pentylen, bedeutet und $R_3$ ein über ein Ringkohlenstoff- oder Ringstickstoffatom gebundenes, gegebenenfalls durch Niederalkyl, insbesondere $C_1$-$C_4$-Alkyl, wie Methyl, substituiertes Imidazolyl, wie Imidazol-1-yl, Imidazol-5-yl, 1-Methyl-imidazol-2-yl oder 4-Methyl-imidazol-5-yl, oder Pyridyl, wie 2- oder 3-Pyridyl, bedeutet, oder worin $R_1$ Hydroxy ist und $R_2$ für $-A-R_3$ steht, worin A die oben angegebene Bedeutung hat und $R_3$ Amino, Di-$C_1$-$C_5$-alkylamino, wie Dimethyl-, N-Methyl-N-n-propylamino oder N-Methyl-N-n-pentylamino, N-$C_1$-$C_4$-Alkyl-N-phenyl-$C_1$-$C_5$-alkylamino, wie N-Methyl-N-(3-phenylpropyl)-amino oder N-Methyl-N-(5-phenylpentyl)-amino, N-$C_1$-$C_4$-Alkyl-N-pyridyl-$C_1$-$C_4$-alkylamino, wie N-Methyl-N-[3-(2-pyridyl)-propyl]-amino, $C_4$-$C_6$-Alkylenamino, welches gegebenenfalls durch gegebenenfalls Halogen, wie Chlor, enthaltendes Phenyl, wie 4-Chlorphenyl, substituiert ist, z.B. Piperidin-1-yl, welches gegebenenfalls, insbesondere in Position 4, durch Phenyl substituiert ist, oder Pyrrolidin-1-yl, welches gegebenenfalls, insbesondere in Position 3, durch 4-Chlorphenyl substituiert ist, oder 1,5-Di-$C_1$-$C_4$-alkyl-, wie 1,5-Dimethyl-3-aza-bicyclo[3.1.1]hept-3-yl bedeutet, oder worin $R_1$ Hydroxy ist und $R_2$ für $-A-R_3$ steht, worin A $C_1$-$C_4$-Alkylen, insbesondere Methylen, bedeutet und $R_3$ Wasserstoff ist, eingearbeitet wird.

6. Verfahren gemäss Anspruch 1, dadurch gekennzeichnet, dass ein pharmazeutisch verwendbares Methandiphosphonsäure-Derivat der Formel I oder ein Salz davon, worin $R_1$ Wasserstoff oder Hydroxy bedeutet und $R_2$ für $-A-R_3$ steht, worin A $C_1$-$C_7$-Alkylen, wie Methylen, Ethylen, Propylen oder Pentylen, bedeutet und $R_3$ N-$C_3$-$C_7$-Cycloalkylamino, wie N-Cycloheptylamino, oder N-$C_1$-$C_4$-Alkyl-N-phenylthio-$C_1$-$C_4$-alkyl, wie N-Methyl-N-(2-phenylthioethyl)-amino, bedeutet, eingearbeitet wird.

7. Verfahren gemäss Anspruch 1, dadurch gekennzeichnet, dass ein pharmazeutisch verwendbares Methandiphosphonsäure-Derivat der Formel I oder ein Salz, insbesondere Alkalimetall-, z.B. Natriumsalz, davon, worin $R_1$ Hydroxy ist und $R_2$ für $-A-R_3$ steht, worin A $C_2$-$C_7$-Alkylen, wie Ethylen, Propylen oder Pentylen, bedeutet und $R_3$ Amino, Di-$C_1$-$C_4$-alkylamino, wie Dimethyl- oder N-Methyl-N-n-propylamino, oder N-$C_1$-$C_4$-Alkyl-N-phenyl-$C_1$-$C_4$-alkylamino, wie N-Methyl-N-(3-phenylpropyl)-amino, bedeutet, eingearbeitet wird.

8. Verfahren gemäss Anspruch 1, dadurch gekennzeichnet, dass ein pharmazeutisch verwendbares Methandiphosphonsäure-Derivat der Formel I oder ein Salz, insbesondere Alkalimetall-, z.B. Natriumsalz, davon, worin $R_1$ Hydroxy ist und $R_2$ für $-A-R_3$ steht, worin A $C_1$-$C_7$-Alkylen, insbesondere $C_1$-$C_4$-Alkylen, wie Methylen, bedeutet und $R_3$ ein über ein Ringkohlenstoff- oder Ringstickstoffatom gebundenes, gegebenenfalls durch $C_1$-$C_4$-Alkyl, wie Methyl, substituiertes Imidazolyl, wie Imidazol-1-yl, Imidazol-5-yl, 1-Methyl-imidazol-2-yl oder 4-Methyl-imidazol-5-yl, bedeutet eingearbeitet wird.

9. Verfahren gemäss Anspruch 1, dadurch gekennzeichnet, dass ein pharmazeutisch verwendbares Methandiphosphonsäure-Derivat ausgewählt aus folgenden Verbindungen der Formel I: 4-Amino-1-hydroxy-butan-1,1-diphosphonsäure, 6-Amino-1-hydroxy-hexan-1,1-diphosphonsäure, 3-(N-Methyl-N-n-pentyl-amino)-1-hydroxy-propan-1,1-diphosphonsäure, 3-[N-(3-Phenylpropyl)-N-methyl-amino]-1-hydroxy-propan- 1,1-diphosphonsäure, 3-(N-Methyl-N-5-phenyl-pentyl-amino)-1-hydroxypropan-1,1-diphosphonsäure, 3-[N-Methyl-N-3-(2-pyridyl)-propyl-amino]-1-hydroxy-propan-1,1-diphosphonsäure, 1-Hydroxy-3-[N-methyl-N-(3-phenoxypropyl)-amino]-propan-1,1-diphosphonsäure, 1-Hydroxy-3-[N-methyl-N-(2-phenoxyethyl)-amino]-propan-1,1-diphosphonsäure, 4-(4-Phenyl-piperidin-1-yl)-1-hydroxy-butan-1,1-diphosphonsäure, 1-Hydroxy-3-(1-piperidino)-propan-1,1-di-phosphonsäure, 1-Hydroxy-3-[3-(4-chlorphenyl)-pyrrolidin-1-yl]-propan-1,1-diphosphonsäure, 1-Hydroxy-2-(imidazol-1-yl)-ethan-1,1-diphosphonsäure, 2-(1-Methyl-imidazol-2-yl)-ethan-1,1-diphosphonsäure, 1-Hydroxy-2-(4-methyl-imidazol-5-yl)-ethan-1,1-di-phosphonsäure, 1-Hydroxy-2-(imidazol-5-yl)-ethan-1,1-diphosphonsäure, 1-Hydroxy-2-(3-pyridyl)-ethan-1,1-diphosphonsäure, 2-(2-Pyridyl)-ethan-1,1-diphosphonsäure, 1-[(5-n-Butyl-2-thiazolyl)-amino]-methan-1,1-diphosphonsäure, 1-[(5-Methyl-2-thiazolyl)-amino]-methan-1,1-diphosphonsäure, 1-[(2-Thiazolyl)-amino]-methan-1,1-diphosphonsäure, 1-Hydroxy-ethan-1,1-diphosphonsäure,1-(4-Chlor-phenylthio)-methan-1,1-diphosphonsäure, 1,1-Dichlormethan-1,1-diphosphonsäure oder 3-(1,5-Dimethyl-3-aza-bicyclo[3.1.1]hept-3-yl)-1-hydroxy-propan-1,1-diphosphonsäure oder ein pharmazeutisch verwendbares Salz davon eingearbeitet wird.

10. Verfahren gemäss Anspruch 1, dadurch gekennzeichnet, dass ein pharmazeutisch verwendbares Methandiphosphonsäure-Derivat ausgewählt aus folgenden Verbindungen der Formel I: 1-[(5-Ethyl-2-thiazolyl)-amino]-methan-1,1-diphosphonsäure, 3-[N-(2-Phenylethyl)-N-methyl-amino]-1-hydroxy-propan-1,1-diphosphonsäure, 3-[N-(2-Phenylthioethyl)-N-methyl-amino]-1-hydroxy-propan-1,1-diphosphonsäure, 1-Hydroxy-3-(pyrrolidin-1-yl)-propan-1,1-diphosphonsäure, 2-(N-Cycloheptylamino)-ethan-1,1-diphosphonsäure oder ein pharmazeutisch verwendbares Salz davon eingearbeitet wird.

11. Verfahren gemäss Anspruch 1, dadurch gekennzeichnet, dass 1-Hydroxy-2-(imidazol-1-yl)-ethan-1,1-diphosphonsäure oder ein pharmazeutisch verwendbares Salz davon eingearbeitet wird.

12. Verfahren gemäss einem der Ansprüche 1-11 zur Herstellung von topisch applizierbaren pharmazeutischen Präparaten enthaltend ein pharmazeutisch verwendbare Methandiphophonsäure-Derivat gemäss einem der Ansprüche 1-11 oder ein Salz davon in Form von Cremen, Gelen, Salben, Lotionen, Pasten, Schäumen, Tinkturen, Mikroemulsionen oder Lösungen.

13. Verfahren gemäss einem der Ansprüche 1-11 zur Herstellung von topisch applizierbaren pharmazeutischen Präparaten enthaltend ein pharmazeutisch verwendbare Methandiphophonsäure-Derivat gemäss einem der Ansprüche 1-11 oder ein Salz davon in Form mehrschichtiger therapeutischer Systeme für die transdermale Applikation.

14. Verfahren gemäss Anspruch 13 zur Herstellung von topisch applizierbaren pharmazeutischen Präparaten enthaltend ein pharmazeutisch verwendbare Methandiphophonsäure-Derivat gemäss einem der Ansprüche 1-11 oder ein Salz davon in Form mehrschichtiger therapeutischer Systeme für die transdermale Applikation, worin die mehrschichtigen therapeutischen Systeme für die transdermale Applikation folgende Bestandteile enthalten:

(1) eine geschlossene und für die Bestandteile der nachfolgenden Schichten der Wirkstoffzubereitung undurchlässige Deckfolie,

(2) ein an die Deckfolie (1) einseitig anschliessendes Reservoir für den Wirkstoff, falls dieser nicht in die Kleberschicht eingearbeitet ist,

(3) eine Kleberschicht und

(4) eine abziehbare Schutzfolie.

15. Verfahren gemäss Anspruch 14 zur Herstellung von topisch applizierbaren pharmazeutischen Präparaten enthaltend ein pharmazeutisch verwendbare Methandiphophonsäure-Derivat gemäss einem der Ansprüche 1-11 oder ein Salz davon in Form mehrschichtiger therapeutischer Systeme für die transdermale Applikation vom Matrix- oder vom Membran-Typ.